(19) Europäisches Patentamt
European Patent Office
Office européen des brevets

(11) Publication number: 0 103 445
A2

(12) **EUROPEAN PATENT APPLICATION**

(21) Application number: 83305093.3

(51) Int. Cl.³: C 07 C 177/00

(22) Date of filing: 02.09.83

(30) Priority: 03.09.82 JP 152809/82
21.09.82 JP 163082/82

(43) Date of publication of application: 21.03.84
Bulletin 84/12

(84) Designated Contracting States: CH DE FR GB IT LI SE

(71) Applicant: TEIJIN LIMITED, 11, 1-Chome,
Minamihonmachi Higashi-ku, Osaka-shi Osaka (JP)

(72) Inventor: Noyori, Ryoji, 135-417, Umemori Shinden
Nisshin-cho, Aichi-gun Aichi-ken (JP)
Inventor: Suzuki, Masaaki, 2-66, Yata-machi Higashi-ku,
Nagoya-shi Aichi-ken (JP)
Inventor: Kurozumi, Seizi, 1-2-28, Higashitokura,
Kokubunji-shi Tokyo (JP)
Inventor: Kawagishi, Toshio, Mukoda
Apt. 3-11538 Mukoda, Minamiashigara-shi
Kanagawa-ken (JP)

(74) Representative: Myerscough, Philip Boyd et al, J.A.Kemp
& Co. 14, South Square Gray's Inn, London, WC1R 5EU
(GB)

(54) **Process for production of prostaglandins E or F, and novel 7-(carbo- or thiocarbo-acyloxy) prostaglandins E or F.**

(57) A novel process for the production of prostaglandins E or F which comprises subjecting a 7-(thio-carboacyloxy)prostaglandin to selective deoxygenation reaction to split off the 7-(thioacyloxy) group, and thereafter, as required, subjecting the reaction product to reduction, deprotection and/or hydrolysis.

The present invention also provide a 7-(carboacyloxy or thiocarboacyloxy)prostaglandin E or F represented by the formula:

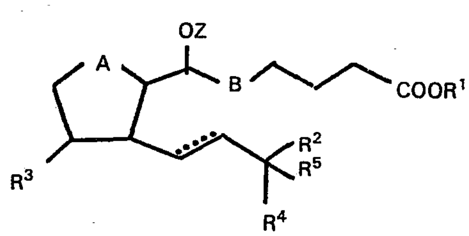

wherein
$R^1$ represents H, $C_{1-10}$ alkyl, or tri($C_{1-7}$ hydrocarbon)silyl,
$R^2$ represents a $C_{1-10}$ alkyl 5- or 6-membered cycloalkyl which may be substituted,
$R^5$ represents H or $CH_3$,
each of $R^3$ and $R^4$ is H, OH or a protected hydroxyl,
the symbol .... represents a single or double bond,
A represents $>C=O$ or $>CH-OH$,
B represents $-CH_2CH_2-$, $-CH=CH-$ or $-C\equiv C-$, and
Z represents carboacyl or thiocarboacyl.

EP 0 103 445 A2

TITLE: "PROCESS FOR PRODUCTION OF PROSTAGLANDINS E OR F, AND NOVEL 7-(CARBO- OR THIOCARBO-ACYLOXY) PROSTAGLANDINS E OR F"

This invention relates to a novel process for the production of prostaglandins E and F (to be referred to as PGE and PGF, respectively), and to novel 7-(carbo- or thiocarbo-acyloxy)PGE or PGF.

Natural $PGE_2$ and $PGF_2\alpha$, which are typical examples of PGE and PGF, have the activity of contracting smooth muscles of the uterus, and are used as most useful labor inducing agents. On the other hand, natural $PGE_1$, one of 1-type prostaglandins, has unique biological activities such as platelet aggregation inhibiting activity and hypotensive activity and has recently been used as a peripheral circulation treating agent in the filed of medical therapy.

A number of methods have been known for the production of these PGE and PGF compounds [see J. B. Bindra et al., Prostaglandin Synthesis, Academic Press (1977)]. Typical among them are as follows:

(i) A method comprising biosynthesis from arachidonic acid or bishomo-γ-linolenic acid [see B. Samuelson et al., Angev. Chem. Int. Ed. Engl., 4, 410 (1965)].

(ii) A method which goes through the Corey lactone which is an important intermediate [see E. J. Corey et al., J. Amer. Chem. Soc., 92, 397 (1970)].

(iii) A method which does through a 2-substituted-2-cycloprentenone compound which is an important intermediate [C. J. Sih et al., J. Amer. Chem. Soc., 97, 865 (1975)].

(iv) A method which comprises selectively reducing 5,6-dehydro $PGE_2$ or $PGF_2\alpha$ [see C. H. Lin et al., Prostaglandin, 11, 377 (1976)].

According to the biosynthetic method (i), the yield of the desired product from the starting arachidonic acid or bishomo-γ-linolenic acid is extremely low, and therefore, the resulting PGE or PGF is difficult to

obtain in purified form from the reaction mixture containing by-products. On the other hand, the processes (ii) to (iv) based on chemical synthesis require many steps in obtaining the starting materials. Even if such a starting material is easily obtained, these processes still have the defect that the yield of the desired prostaglandin E from the starting material is very low.

In an attempt to overcome these difficulties, methods comprising conjugated addition reaction of a 2-cyclopentenone compound followed by a step of trapping the enolate were recently proposed to synthesize the PG skeleton [see G. Stork, et al., M. Amer. Chem. Soc., 97, 6062 (1975), and K. G. Untch et al., J. Org. Chem. 44, 3755].

These attempts, however, require multiple steps in which the enolate is trapped by using formaldehyde or trimethylsilyl chloride which is a low-molecular-weight compound, and through the resulting important intermediate, the PG skeleton is synthesized by chemical synthesis. Moreover, the total yield of the final product obtained by such a method is low.

It is an object of this invention therefore to provide a process for producing prostaglandins E or F with fewer steps than any chemical synthetic processes known heretofore.

Another object of this invention is to provide a process for producing prostaglandins E or F in higher yields than in any conventional known processes.

Still another object of this invention is to provide an industrially very advantageous process for producing prostaglandins E or F using novel 7-(thiocarboacyloxy)prostaglandins E or F as starting materials.

Yet another object of this invention is to provide novel 7-(thiocarboacyloxy)prostaglandins E or F which are useful as starting materials in the above process of this invention, and to provide novel 7-(carboacyloxy)prostaglandins E or F which have useful pharmacological activities.

A further object of this invention is to provide a process which comprises using as a starting material a 7-hydroxyprostaglandin E obtained in a high yield by a one-step reaction from protected 4-hydroxy-2-cyclopentenone, converting the hydroxy group at the 7-position of the starting material to a thiocarboacyloxy group and then selectively removing it, and if desired, submitting the product to functional transformation whereby PGE or PGF is produced in high yields from the starting material in a very much reduced number of reaction steps.

These objects and advantages of this invention are achieved in accordance with this invention by a novel process for the production of prostaglandins E or F represented by the following formula (I)

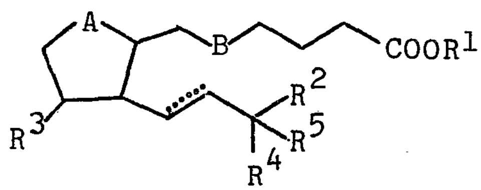 ........... (I)

wherein

$R^1$ represents a hydrogen atom, a $C_{1-10}$ alkyl group or a tri($C_{1-7}$ hydrocarbon)silyl group,

$R^2$ represents an alkyl group having 1 to 10 carbon atoms which may be substituted, or a 5- or 6-membered cycloalkyl group which may be substituted,

$R^5$ represents a hydrogen atom or a methyl group,

$R^3$ and $R^4$ are identical or different and each represents a hydrogen atom, a hydroxyl group or a protected hydroxyl group,

the symbol ........ represents a single or double bond,

A represents a carbonyl group (>C=O) or a hydroxymethylene group (>CH-OH), and

B represents $-CH_2CH_2$, -CH=CH- or -C≡C-,

which comprises subjecting a 7-(thiocarboacyloxy)-prostaglandin represented by the following formula (II)

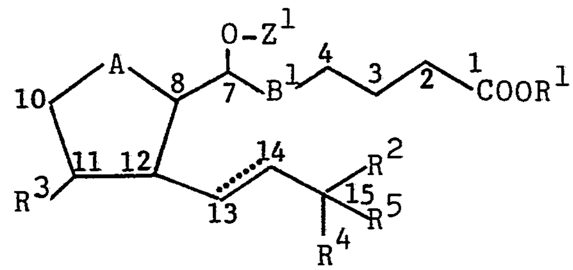
....... (II)

wherein

$R^1$, $R^2$, $R^3$, $R^4$, $R^5$ and A are as defined,

$B^1$ represents -CH=CH- or -C≡C-, and

$Z^1$ represents a thiocarboacyl group,

to selective deoxygenation reaction, and thereafter, as required, subjecting the reaction product to reduction, deprotection and/or hydrolysis.

In the present application, the terms "carboacyl and thiocarboacyl" denote acyl groups derived from carboxylic acids and acyl groups derived from thiocarboxylic acids, respectively.

In formulae (I) and (II), $R^1$ represents a hydrogen atom, an alkyl group having 1 to 10 carbon atom, or a tri($C_{1-7}$ hydrocarbon)silyl group. Examples of the alkyl group having 1 to 10 carbon atoms are linear or branched $C_1$-$C_{10}$ alkyl groups such as methyl, ethyl, n-propyl, iso-propyl, n-butyl, sec-butyl, tert-butyl, n-pentyl, n-hexyl, n-heptyl, n-octyl, n-nonyl and n-decyl groups.

In the tri($C_{1-7}$ hydrocarbon)silyl group, the hydrocarbon moiety with 1 to 7 carbon atoms may include linear or branched alkyl groups such as methyl, ethyl, n-propyl, n-butyl, tert-butyl, n-pentyl, n-hexyl and n-heptyl groups, and aryl or aralkyl groups such as phenyl, tolyl and benzyl groups. Examples of the tri-($C_{1-7}$ hydrocarbon)silyl group are tri($C_1$-$C_4$ alkyl)silyl groups such as trimethylsilyl triethylsilyl and tert-butyldimethylsilyl groups, diphenyl ($C_1$-$C_4$ alkyl)silyl groups such as a tert-butyldiphenylsilyl group, and a tribenzylsilyl group.

$R^2$ represents an alkyl group having 1 to 10 carbon atoms which may be substituted, or a 5- or 6-membered cycloalkyl group which may be substituted. Examples of the alkyl group having 1 to 10 carbon atoms include those exemplified hereinabove for $R^1$ and also include 2-hexyl and 2-methylhexyl groups. Of these, n-pentyl, n-hexyl, 2-hexyl and 2-methylhexyl groups are preferred. Examples of the 5- or 6-membered cycloalkyl group are cyclopentyl and cyclohexyl groups. Such a $C_1$-$C_{10}$ alkyl group or 5- or 6-membered cycloalkyl group may be substituted by a lower alkyl group such as methyl or ethyl (excepting $R^2$ being the $C_{1-10}$ alkyl group), a lower alkoxy group such as methoxy or ethoxy, a cycloalkyl group such as cyclopentyl or cyclohexyl, and such a substituent as ethynyl, phenyl, phenoxy, trifluorophenyl or trifluoromethyl.

$R^5$ is a hydrogen atom or a methyl group.

$R^3$ and $R^4$ are identical or different, and each represents a hydrogen atom, a hydroxyl group or a protective group for the hydroxyl group. Examples of the protective group are tri($C_{1-7}$ hydrocarbon)silyl groups, or groups capable of forming an acetal linkage with the oxygen atom of the hydroxyl group. Examples of the tri($C_{1-7}$ hydrocarbon)silyl group may be the same as those exemplified hereinabove with regard to $R^1$. Examples of the groups capable of forming an acetal linkage together with the oxygen atom of the hydroxyl group include methoxyethyl, 1-ethoxyethyl, 2-methoxy-2-propyl, 2-ethoxy-2-propyl, (2-methoxyethoxy) methyl, benzyloxymethyl, 2-tetrahydropyranyl, 2-tetrahydrofuranyl, and 6,6-dimethyl-3-oxa-2-oxo-bicyclo[3.1.0]hex-4-yl groups. Of these, the tert-butyldimethylsilyl, 1-ethoxyethyl, 2-methoxy-2-propyl, (2-methoxyethoxy)methyl, and 2-tetrahydropyranyl groups are preferred.

A represents a carbonyl group (>C=O) or a hydroxy methylene group (>CH-OH).

The symbol ....... represents a single or double bond.

$Z^1$ represent a thiocarboacyl group.

Examples of the thiocarboacyl group include those represented by the formula

$$-\underset{\overset{\|}{S}}{C}-X-G$$

wherein X represents a bond, an oxygen atom, a sulfur atom or -NHCO-, and G represents a substituted or unsubstituted $C_{1-6}$ alkyl group, phenyl or imidazolyl group. The substituent on the substituted $C_{1-6}$ alkyl, phenyl or imidazolyl group may, for example, be a $C_{1-6}$ alkyl group (excepting G being the $C_{1-6}$ alkyl group), a $C_{1-6}$ alkoxy group, a halogen atom or a nitro group.

Examples of the group $Z^1$ include methyl(thiocarbonyl), ethyl(thiocarbonyl), propyl(thiocarbonyl), hexyl(thiocarbonyl), trifluoromethyl(thiocarbonyl), phenyl(thiocarbonyl), tolyl(thiocarbonyl), p-chlorophenyl(thiocarbonyl), p-methoxyphenyl(thiocarbonyl), 2,4-dimethoxyphenyl(thiocarbonyl), p-nitrophenyl(thiocarbonyl), imidazo(thiocarbonyl), methoxy(thiocarbonyl), ethoxy(thiocarbonyl), hexoxy(thiocarbonyl), phenoxy(thiocarbonyl), p-methoxyphenoxy(thiocarbonyl), p-nitrohexoxy-(thiocarbonyl), methylthio(thiocarbonyl), phenylthio-(thiocarbonyl), imidazothio(thiocarbonyl), acetylamino-(thiocarbonyl), propionylamino(thiocarbonyl), benzoylamino(thiocarbonyl), and p-methoxybenzoylamino(thiocarbonyl). Of these, phenyl(thiocarbonyl), imidazo-(thiocarbonyl), phenoxy(thiocarbonyl), methylthio(thiocarbonyl) and benzoylamino(thiocarbonyl) are preferred.

In formula (II), $B^1$ represents -CH=CH- or -C≡C.

In formula (I), B represents $-CH_2CH_2-$, -CH=CH- or -C≡C-.

For the sake of convenience, the starting 7-(thiocarboacyloxy)prostaglandins of formula (II) can be classified, according to the definition of A, into 7-(thiocarboacyloxy)prostaglandins E of the following formula (II)-1

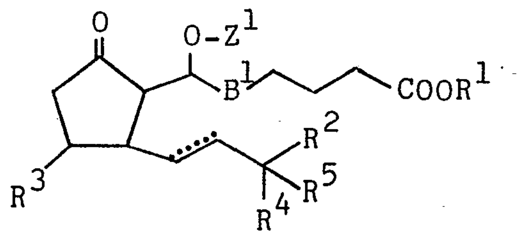

.......... (II)-1

wherein $R^1$, $R^2$, $R^3$, $R^4$, $R^5$, $B^1$, $Z^1$ and the symbol ......... are the same as in formula (II), and 7-(thiocarboacyloxy)prostaglandines F of the following formula (II)-2

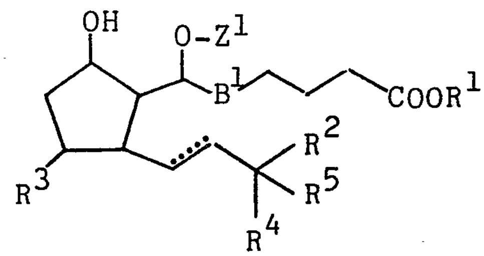

.......... (II)-2

wherein $R^1$, $R^2$, $R^3$, $R^4$, $R^5$, $B^1$, $Z^1$ and the symbol ......... are the same as in formula (II).

The starting materials of formula (II) are 7-(thiocarboacyloxy) derivatives of the corresponding 7-hydroxyprostaglandins, and therefore, those skilled in the art will easily understand examples of the starting materials of formula (II) from those of the corresponding 7-hydroxyprostaglandins.

Examples of the 7-hydroxy compounds corresponding to the 7-(thiocarboacyloxy)prostaglandins E of formula (II)-1 are given below.

(200) 7-Hydroxy $PGE_2$,

(202) 7-Hydroxy-16-methyl $PGE_2$,

(204) 7-Hydroxy-16,16-dimethyl $PGE_2$,

(206) 7-Hydroxy-20-methyl $PGE_2$,

(208) 7-Hydroxy-17,20-dimethyl $PGE_2$,

(210) 7-Hydroxy-16,17,18,19,20-pentanor-15-cyclopentyl $PGE_2$,

(212) 7-Hydroxy-16,17,18,19,20-pentanor-15-cyclohexyl $PGE_2$,

(214) 7-Hydroxy-15-methyl $PGE_2$,

(216) 7-Hydroxy-17,17,20-trimethyl $PGE_2$,

(218) 7-Hydroxy-17,18,19,20-tetranor-16-cyclohexyl $PGE_2$,

(220) 7-Hydroxy-17,18,19,20-tetranor-16-phenoxy-$PGE_2$,

(222) 7-Hydroxy-5,6-dehydro-$PGE_2$,

(224) 7-Hydroxy-5,6-dehydro-16-methyl $PGE_2$,

(226) 7-Hydroxy-5,6-dehydro-16,16-dimethyl $PGE_2$,

(228) 7-Hydroxy-5,6-dehydro-20-methyl $PGE_2$,

(230) 7-Hydroxy-5,6-dehydro-17,20-dimethyl $PGE_2$,

(232) 7-Hydroxy-5,6-dehydro-16,17,18,19,20-pentanor-15-cyclopentyl $PGE_2$,

(234) 7-Hydroxy-5,6-dehydro-16,17,18,19,20-pentanor-15-cyclohexyl $PGE_2$,

(236) 7-Hydroxy-5,6-dehydro-15-methyl $PGE_2$,

(238) 7-Hydroxy-5,6-dehydro-17,17,20-trimethyl $PGE_2$,

(240) 7-Hydroxy-5,6-dehydro-18-oxa $PGE_2$,

(242) 7-Hydroxy-5,6-dehydro-17,18,19,20-tetranor-16-cyclopentyl $PGE_2$.

Examples of the 7-hydroxy compounds corresponding to the 7-(thiocarboacyloxy)prostaglandins F represented by formula (II)-2 are given below.

(250) 7-Hydroxy $PGF_2$,

(252) 7-Hydroxy-16-methyl $PGF_2$,

(254) 7-Hydroxy-16,16-dimethyl $PGF_2$,

(256) 7-Hydroxy-20-methyl $PGF_2$,

(258) 7-Hydroxy-17,20-dimethyl $PGF_2$,

(260) 7-Hydroxy-16,17,18,19,20-pentanor-15-cyclopentyl $PGF_2$,

(262) 7-Hydroxy-16,17,18,19,20-pentanor-15-cyclohexyl $PGF_2$,

(264) 7-Hydroxy-15-methyl $PGF_2$,

(266) 7-Hydroxy-17,17,20-trimethyl $PGF_2$,

(268) 7-Hydroxy-17,18,19,20-tetranor-16-cyclohexyl $PGF_2$,

(270) 7-Hydroxy-17,18,19,20-tetranor-16-phenoxy-$PGF_2$,

(272) 7-Hydroxy-5,6-dehydro-$PGF_2$,

(274) 7-Hydroxy-5,6-dehydro-16-methyl $PGF_2$,

(276) 7-Hydroxy-5,6-dehydro-16,16-dimethyl $PGF_2$,

(278) 7-Hydroxy-5,6-dehydro-20-methyl $PGF_2$,

(280) 7-Hydroxy-5,6-dehydro-17,20-dimethyl $PGF_2$,

(282) 7-Hydroxy-5,6-dehydro-16,17,18,19,20-pentanor-15-cyclopentyl $PGF_2$,

(284) 7-Hydroxy-5,6-dehydro-16,17,18,19,20-pentanor-15-cyclohexyl $PGF_2$,

(286) 7-Hydroxy-5,6-dehydro-15-methyl $PGF_2$,

(288) 7-Hydroxy-5,6-dehydro-17,17,20-trimethyl $PGF_2$,

(290) 7-Hydroxy-5,6-dehydro-18-oxa $PGF_2$,

(292) 7-Hydroxy-5,6-dehydro-17,18,19,20-tetranor-16-cyclopentyl $PGF_2$.

According to the process of this invention, the 7-(thiocarboacyloxy)prostaglandin of formula (II) [including formulae (II)-1 and (II-2] is first subjected to selective deoxygenation reaction. It should be understood that the selective deoxygenation reaction denotes a reaction of selectively converting only the hydroxyl group at the 7-position (actually, the thiocarboacyloxy group) among the hydroxyl groups (or protected hydroxyl groups) present on the prostaglandin skeleton into a hydrogen atom.

Methods *per se* of converting a hydroxyl group to a hydrogen atom have been known in the chemistry of sugars or steroids [see, for example, D. H. R. Barton et al., J. Chem. Soc., Perkins I, 1574 (1975); M. J. Robins et al., J. Amer. Chem. Soc., 103, 932 (1981); and R. R. Rasmussen, J. Org. Chem., 46, 4843 (1981)]. It is no exaggeration to say however that according to the process of this invention, unexpected results can be achieved, as can be seen from Examples given hereinbelow, in that only the hydroxyl group at the 7-position of the prostaglandin skeleton can be converted to a hydrogen

atom at a very high conversion.

The selective deoxygenation reaction in accordance with this invention is carried out in the presence of an organic tin compound of the following formula

$$(R^6)_3SnH$$

wherein $R^6$ represents a $C_{1-10}$ alkyl or phenyl group.

Preferably, this reaction is carried out in the further presence of a radical generator.

Examples of the organic tin compound include trimethyltin hydride, triethyltin hydride, tri-n-butyltin hydride, tri-n-hexyltin hydride, tri-n-octyltin hydride, tri-n-decyltin hydride, triphenyltin hydride and diphenylmonomethyltin hydride. Of these, tri-n-butyltin hydride and triphenyltin hydride are used preferably.

Examples of suitable radical generators for use in this invention are α,α-azobisisobutyronitrile, bis-tert-butyl peroxide, tert-butyl hydroperoxide, cumene hydroperoxide, benzoyl peroxide and lauroyl peroxide.

Stoichiometrically, the organic tin compound react with the 7-(thiocarboacyloxy)prostaglandin in equimolar proportions. Usually, the organic tin compound is used in an amount of 0.5 to 50 moles, preferably 0.8 to 30 moles, per mole of the 7-(thiocarboacyloxy)-prostaglandin.

If desired, the reaction can be also be carried out in an inert organic solvent. Preferably, such an inert organic solvent may be an aromatic hydrocarbon such as benzene or toluene, an aliphatic hydrocarbon such as pentane or hexane, or an ether such as diethyl ether or tetrahydrofuran. The end point of the reaction can be determined by monitoring the disappearance of the starting compound by, for example, thin-layer chromatography. For example, when the reaction temperature is 50 °C, the reaction ends in 0.5 to 12 hours.

Thus, according to the selective deoxygenation reaction, a prostaglandin E or F of the following formula (I)'

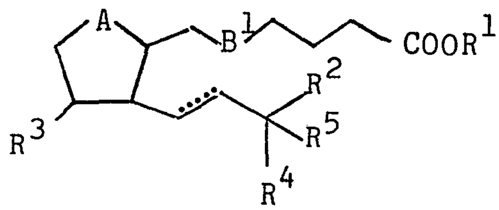

........... (I)'

wherein $R^1$, $R^2$, $R^3$, $R^4$, $R^5$, A, $B^1$ and the symbol .... are as defined with regard to formula (II),

is produced from the starting material of formula (II).

To separate the resulting prostaglandin E or F from the reaction mixture after the reaction, the reaction mixture is treated by ordinary means, for example extraction, washing with water, drying, chromatography, etc.

A comparison of formula (I)' with formula (I) representing the prostaglandin E or F as the final desired product of the process of this invention shows that $B^1$ in formula (I)' is B in formula (I), and otherwise, formula (I)' is the same as formula (I). It will be seen therefore that the prostaglandins of formula (I)' do not include prostaglandins of formula (I) in which B is $-CH_2CH_2-$, but encompass all other prostaglandins of formula (I).

A prostaglandin of formula (I) in which B is $-CH_2CH_2-$ can be produced by reducing a compound of formula (I)' in which $B^1$ is $-CH=CH-$ or $-C\equiv C-$. To perform the reduction using the compounds of formula (I)' in which a single bond exists between the 13- and 14-positions (namely when the symbol .... is a single bond), there can be employed a hydrogenation method using various hydrogenation catalysts such as a Pd/C catalyst. When the compound of formula (I)' has a double bond between the 13- and 14-positions (namely when the symbol .... is a double bond, i.e. a trans double bond), it is preferred to selectively hydrogenate the compound of

formula (I)' in which $B^1$ is cis -CH=CH- or -C≡C- using, for example, a Lindler catalyst, a palladium-barium sulfate-synthetic quinoline catalyst, or a palladium-carbon catalyst in order to obtain a product having a double bond between the 13- and 14-positions. This selective hydrogenation is usually carried out in an organic solvent such as benzene and cyclohexane, and the reaction time is usually 1 to 15 hours. The reaction temperature is room temperature.

It will be easily understood, of course, that when a product having a reduced double bond between the 13- and 14-positions is to be obtained from the compound of formula (I)' having a double bond between the 13- and 14-positions, a wide range of hydrogenation processes can be employed as in the case of hydrogenating the compound of formula (I)' having a single bond between the 13- and 14-positions.

By carrying out the reducing reaction as above, the process of this invention gives all of the prostaglandins E or F which are included within formula (I) starting from the 7-(thiocarboacyloxy)prostaglandins of formula (II).

The prostaglandins E or F so produced can be subjected to conversion reactions in accordance with the process of this invention. Specifically, by reduction, a compound wherein A is >CH-OH can be produced from a compound wherein A is >C=O, or a compound wherein B is -CH=CH- can be produced from a compound wherein B is -C≡C-. By deprotecting reaction, a compound wherein $R^3$ and $R^4$ are free hydroxyl groups can be produced from a compound wherein $R^3$ and $R^4$ are protected hydroxyl groups. Furthermore, by hydrolysis, a compound wherein $R^1$ is a hydrogen atom can be produced from a compound wherein $R^1$ is a $C_{1-10}$ alkyl group or a tri($C_{1-7}$ hydrocarbon)silyl group.

The reducing reaction of obtaining the compound wherein A is a hydroxymethyl group (>CH-OH) from the compound wherein A is a carbonyl group (>C=O) is carried

out preferably by using a metal borohydride such as sodium borohydride, lithium borohydride, potassium borohydride or zinc borohydride, or a combination of diisobutyl aluminum hydride and 2,6-t-butyl-4-methylphenol. It is especially preferred to use sodium borohydride or a combination of diisobutyl aluminum hydride and 2,6-di-t-butyl-4-methylphenol.

When the metal borohydride is used, an organic solvent such as methanol, ethanol or propanol is preferred as the reaction solvent, and the reaction is usually carried out under ice cooling. When a combination of diisobutyl aluminum hydride and 2,6-di-t-butyl-4-methylphenol is used, toluene is preferred as the reaction solvent, and the reaction is carried out usually at -78 $^{o}C$ for several hours.

The reaction of producing the compound wherein B is -CH=CH- from the compound wherein B is -C≡C- is carried out, for example, by using a Lindlar catalyst or a palladium-barium sulfate-synthetic quinoline catalyst, and stopping the reaction when 1 mole of hydrogen per molecule has been absorbed. This reaction itself is well known, and has the advantage that the compound wherein B is cis -CH=CH- is formed as a main product.

Elimination of the protective groups for the hydroxyl groups can be carried out as shown below. When the protective groups are groups forming acetal linkages with oxygen atoms, the deprotection may be conveniently carried out in a reaction solvent such as water, tetrahydrofuran, ethyl ether, dioxane, acetone or acetonitrile using acetic acid, pyridinium p-toluenesulfonate, a cation exchange resin, etc. as a catalyst. The reaction is carried out usually at -20 $^{o}C$ to +80 $^{o}C$ for a period of 10 minutes to 3 days. When the protective groups are tri($C_{1-7}$ hydrocarbon)silyl groups, the deprotection may be carried out at the same temperature for the same period of time as above in the above-exemplified reaction solvent in the presence of acetic acid, hydrogen fluoride, tetrabutyl ammonium fluoride,

or cesium fluoride, preferably acetic acid and hydrogen fluoride.

Hydrolysis of the ester group ($-COOR^1$ wherein $R^1$ is an alkyl group having 1 to 10 carbon atoms) may be carried out, for example, by treating the prostaglandin E or F, with or without prior deprotection, with an enzyme such as lipase in water or a water-containing solvent at a temperature in the range of 0°C to 40°C for a period of 10 minutes to 24 hours.

The ester group in which $R^1$ is a tri($C_{1-7}$ hydrocarbon)silyl group can be easily converted to a carboxyl group, for example by treatment in an acidic or basic aqueous medium at a temperature of 0°C to +30°C.

According to the process of this invention, a prostaglandin E represented by the following formula (I)-1

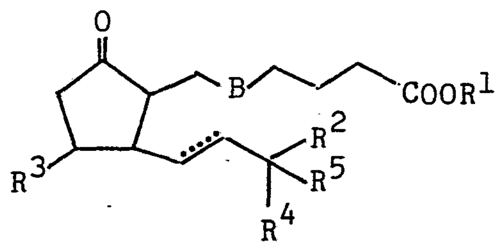

............ (I)-1

wherein $R^1$, $R^2$, $R^3$, $R^4$, $R^5$ and the symbol .... are as defined with regard to formula (II), and B is the same as defined in formula (I),

can be advantageously produced by subjecting the 7-(thiocarboacyloxy)prostaglandin E of formula (II)-1 to selective deoxygenation reaction, and then, as required, subjecting the product to selective reduction of the unsaturated linkage ($B^1$) between the 5- and 6-positions, deprotection and/or hydrolysis.

Likewise, a prostaglandin F represented by the following formula (I)-2 encompassed within formula (I)

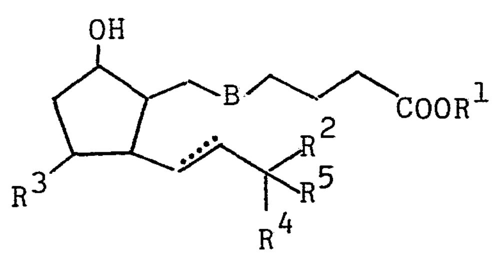

......... (I)-2

wherein $R^1$, $R^2$, $R^3$, $R^4$, $R^5$, B and the symbol .... are the same as defined with regard to formula (I),

can be advantageously produced by subjecting the 7-(thiocarboacyloxy)prostaglandin F represented by formula (II)-2 to selective deoxygenation reaction, or subjecting the 7-(thiocarboacyloxy)prostaglandin E of formula (II)-1 to selective deoxygenation and reuding the oxo group (=O) at the 9-position, and thereafter, as required, subjecting the product to selective reduction of the unsaturated bond ($B^1$) between the 5- and 6-positions, deprotection and/or hydrolysis.

The 7-(thiocarboacyloxy)prostaglandin of formula (II) used as the starting material in the process of this invention can be produced as follows:

The 7-(thiocarboacyloxy)prostaglandin E of formula (II)-1 can be produced by reactgng a 7-hydroxyprostaglandin E of the following formula (a)

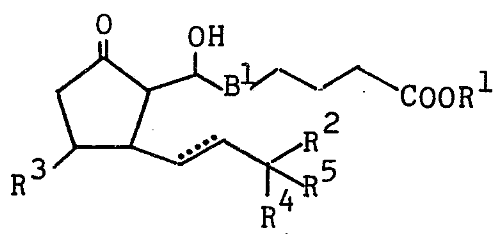 ...........(a)

wherein $R^1$, $R^2$, $R^3$, $R^4$, $R^5$, $B^1$ and the symbol .... are as defined with regard to formula (II), provided that $R^1$ is not a hydrogen atom, and $R^3$ and $R^4$ represent a hydrogen atom or a protected hydroxyl group,

with a halogen compound of the following formula (b)

$X-Z^1$ ...................... (b)

wherein $Z^1$ is as defined with regard to formula (II), and X represents a halogen atom such as chlorine or bromine,

preferably in the presence of a basic compound, and thereafter, as required, subjecting the reaction product to deprotection and/or hydrolysis.

The basic compound is preferably an amine.

Examples of the amine are 4,4-dimethyl-aminopyridine, pyridine, triethylamine, diisopropylcyclohexylamine, isopropyldimethylamine and diisopropylethylamine. Of these, 4,4-dimethylaminopyridine and pyridine are preferred.

The halogen compound of formula (b) is used in an amount of 0.5 to 10 equivalents, preferably 2 to 3 equivalents, per equivalent of the starting compound of formula (a). The basic compound is used in an amount of 1 to 10 equivalents, prefetably 4 to 6 equivalents, per equivalent of the starting compound of formula (a).

The reaction temperature is 0 to 50 $^{o}$C, preferably 10 to 40 $^{o}$C. The end point of the reaction can be determined by tracing the disappearance of the starting compound by thin-layer chromatography, for example. The reaction time is usually 0.5 to 10 hours. To make the reaction proceed smoothly, a solvent may be used. Examples of the solvent include halogenated hydrocarbons such as dichloromethane, chloroform and carbon tetrachloride, ethers such as diethyl ether and tetrahydrofuran, and hydrocarbons such as benzene, toluene, pentane, hexane and cyclohexane. Dichloromethane is preferred.

The reaction product can be purified and recovered by treating the reaction mixture by ordinary means, for example extraction, washing with water, drying, chromatography, etc.

The 7-(thiocarboacyloxy)prostaglandin F of formula (II)-2 encompassed within formula (II) can be produced by reducing the 7-(thiocarboacyloxy)prostaglandin E of formula (II)-1 obtained as above, by a method known *per se* to convert the carbonyl group at the 9-position to a hydroxymethylene group, and thereafter, as required, subjecting the product to deprotection and/or hydrolysis [see S. Iguchi et al., Bull, Chem. Soc., Japan, 54, 3033 (1981); G. P. Pollini et al., J. Org. Chem., 45, 3141 (1980); and K. G. Untch et al., J. Org. Chem., 44, 3755 (1979)]. Examples of reducing agents which can be preferably used in this reducing reaction include

sodium borohydride, trialkyl($C_1$-$C_4$) lithium borohydrides, trialkyl($C_1$-$C_4$) potassium borohydrides, diisobutyl aluminum hydride, and diisobutyl aluminum 2,6-di-t-butyl-4-methylphenoxide.

The resulting product can be purified and recovered by treating the reaction mixture by ordinary means, for example extraction, washing with water, drying, chromatography, etc.

The deprotection and/or hydrolysis in the aforesaid reactions of producing the 7-(thiocarboacyloxy)-prostaglandins of formulae (II)-1 and (II)-2 can be carried out by the same methods as described hereinabove.

The hydroxyprostaglandins of formula (a) are known from European Laid-Open Patent Publication No. 79733 and U. S. Patent Application Serial No. 438379.

The 7-(thiocarboacyloxy)prostaglandins of formula (II) [including formulae (II)-1 and (II)-2] are novel compounds which are useful themselves because of their useful pharmacological activities, or useful as intermediates for the production of the prostaglandins E or F of formula (I) having useful pharmacological activities. The 7-(thiocarboacyloxy)prostaglandins of formula (II) constitute part of the present invention.

In formula (II), $B^1$ is defined as -CH=CH- or -C≡C-, and the 7-(thiocarboacyloxy)prostaglandins of formula (II) have an unsaturated linkage between the carbon atoms at the 5- and 6-positions.

Investigations of the present inventors have shown that 7-(thiocarboacyloxy)prostaglandins represented by the following formula (II)-a

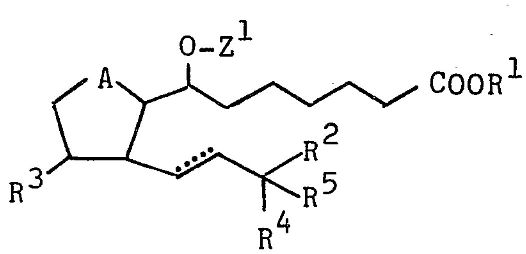

....... (II)-a

wherein $R^1$, $R^2$, $R^3$, $R^4$, $R^5$, A, $Z^1$

and the symbol .... are as defined with regard to formula (II),

in which a single bond exists between carbon atoms at the 5- and 6-positions are novel compounds having useful pharmacological activities like the compounds of formula (II).

The compounds of formula (II)-a can be produced by the aforesaid method of producing the 7-(thiocarboacyloxy)prostaglandin E of formula (II)-1 from the 7-hydroxyprostaglandin E of formula (a) and the halogen compound of formula (b), and by producing the 7-(thiocarboacyloxy)prostaglandin of formula (II)-2 in the same way as in the above method of reducing the compound of formula (II)-1 using a 7-hydroxyprostaglandin $E_1$ of the following formula (a)'

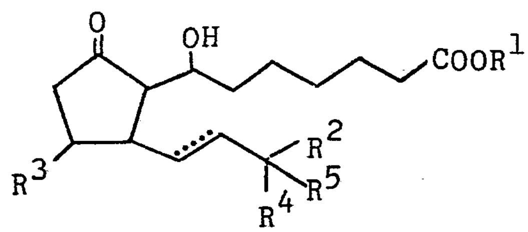

......... (a)'

wherein $R^1$, $R^2$, $R^3$, $R^4$, $R^5$ and the symbol .... are the same as in formula (a),

instead of the compound of formula (a). The compound of formula (a)' can be produced by the method described in the specification of U. S. Patent No. 4,315,032.

Accordingly, a series of novel 7-(thiocarboacyloxy)prostaglandins provided by this invention are expressed by the following formula (II)'

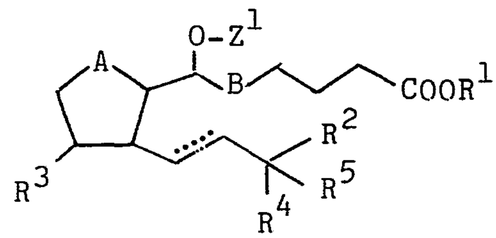

........ (II)'

wherein $R^1$, $R^2$, $R^3$, $R^4$, $R^5$, A, $Z^1$ and the symbol .... are the same as in formula (II), and B is the same as defined in formula (I).

For the sake of convenience, the 7-(thiocarboacyloxy)prostaglandins of formula (II)' can be classfied, according to the definition of A, into 7-(thiocarboacyloxy)prostaglandins E of the following formula (II)'-1

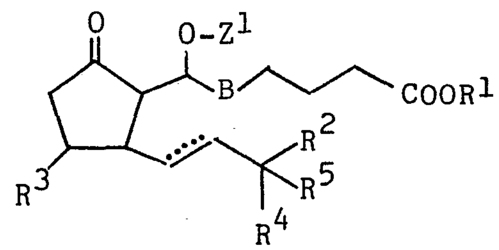

.......... (II)'-1

wherein $R^1$, $R^2$, $R^3$, $R^4$, $R^5$, B, $Z^1$, and the symbol .... are the same as defined in formula (II)',

and 7-(thiocarboacyloxy)prostaglandins F of the following formula (II)'-2

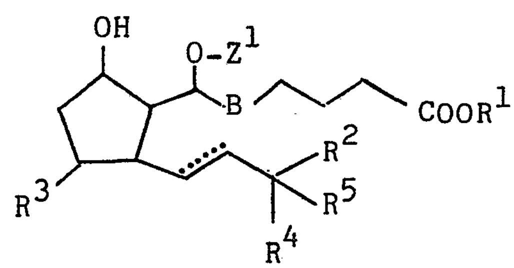

......... (II)'-2

wherein $R^1$, $R^2$, $R^3$, $R^4$, $R^5$, B, $Z^1$ and the symbol .... are the same as defined in formula (II)'.

In formula (II)' [including formulae (II)'-1 and (II)'-2], $R^1$ is preferably a hydrogen atom or a $C_{1-6}$ alkyl group, and $R^2$ is prefefably an unsubstituted $C_{1-10}$ alkyl group, a $C_{1-10}$ alkyl group substituted by phenoxy, trifluoromethyl or trifluoromethylphenoxy, an unsubstituted 5- or 6-membered cycloalkyl group, or a 5- or 6-membered cycloalkyl group substituted by $C_{1-6}$ alkyl, trifluoromethyl, phenoxy or trifluoromethylphenoxy.

$R^3$ and $R^4$ are identical or different, and preferably each represents a hydrogen atom, a hydroxyl group, a tri($C_{1-7}$ hydrocarbon)silyl group, or a group capable of forming an acetal linkage with the oxygen atom

of the hydroxyl group.

$Z^1$ is preferably a thiocarboacyl group represented by the following formula

$$-\underset{\underset{S}{\|}}{C}-X-G$$

wherein X represents a bond, an oxygen atom, a sulfur atom or -NHCO-, and G represents a substituted or unsubstituted $C_{1-6}$ alkyl, phenyl or imidazolyl group.

Examples of the 7-(thiocarboacyloxy)prostaglandins E of formula (II)'-1 are given below.

(300) 7-Phenylthiocarbonyloxy $PGE_1$

(302) 7-Phenylthiocarbonyloxy-16-methyl $PGE_1$

(304) 7-Phenylthiocarbonyloxy-16,16-dimethyl $PGE_1$

(305) 7-Phenylthiocarbonyloxy-20-methyl $PGE_1$

(306) 7-Phenylthiocarbonyloxy-17,20-dimethyl $PGE_1$

(308) 7-Phenylthiocarbonyloxy-16,17,18,19,20-pentanor-15-cyclopentyl-$PGE_1$

(310) 7-Phenoxythiocarbonyloxy-$PGE_1$

(312) 7-Phenoxythiocarbonyloxy-16,16-dimethyl-$PGE_1$

(314) 7-Phenoxythiocarbonyloxy-16,17,18,19,20-pentanor-15-cyclopentyl-$PGE_1$

(316) 7-Methylthiothiocarbonyloxy-$PGE_1$

(318) 7-Methylthiothiocarbonyloxy-17,20-methyl-$PGE_1$

(320) 7-Benzoylaminothiocarbonyloxy-$PGE_1$

(322) 7-Benzoylaminothiocarbonyloxy-16,17,18,19,20-pentanor-15-cyclohexyl-$PGE_1$

(324) 7-Benzoylaminothiocarbonyloxy-15-methyl-$PGE_1$

(326) 7-Phenylthiocarbonyloxy derivative of (200)

(328) 7-Phenylthiocarbonyloxy derivative of (202)

(330) 7-Phenylthiocarbonyloxy derivative of (208)

(332) 7-Phenylthiocarbonyloxy derivative of (212)

(334) 7-Phenylthiocarbonyloxy derivative of (220)

(336) 7-Phenylthiocarbonyloxy derivative of (222)

(338) 7-Phenylthiocarbonyloxy derivative of (226)

(340) 7-Phenylthiocarbonyloxy derivative of (230)

(342) 7-Phenylthiocarbonyloxy derivative of (240)

(344) 7-Phenoxythiocarbonyloxy derivative of (200)

(346) 7-Phenoxythiocarbonyloxy derivative of (204)

(348) 7-Phenoxythiocarbonyloxy derivative of (212)

(350) 7-Phenoxythiocarbonyloxy derivative of (220)

(352) 7-Methylthiothiocarbonyloxy derivative of (200)

(354) 7-Methylthiothiocarbonyloxy derivative of (232)

(356) 7-Benzoylaminothiocarbonyloxy derivative of (214)

(358) 7-Benzoylaminothiocarbonyloxy derivative of (234)

(360) 7-Benzoylaminothiocarbonyloxy derivative of (240)

Examples of the 7-(thiocarboacyloxy)prostaglandins F of formula (II)'-2 are given below.

(400) 7-Phenylthiocarbonyloxy $PGF_1$

(402) 7-Phenylthiocarbonyloxy-16-methyl $PGF_1$

(404) 7-Phenylthiocarbonyloxy-16,16-dimethyl $PGF_1$

(406) 7-Phenylthiocarbonyloxy-17,20-dimethyl $PGF_1$

(408) 7-Phenylthiocarbonyloxy-16,17,18,19,20-pentanor-15-cyclopentyl $PGF_1$

(410) 7-Phenylthiocarbonyloxy-15-methyl $PGF_1$

(412) 7-Phenoxythiocarbonyloxy $PGF_1$

(414) 7-Phenoxythiocarbonyloxy-17,20-dimethyl $PGF_1$

(416) 7-Phenoxythiocarbonyloxy-16,17,18,19,20-pentanor-15- cyclohexyl $PGF_1$

(418) 7-Methylthiothiocarbonyloxy $PGF_1$

(420) 7-Methylthiothiocarbonyloxy-17,20-dimethyl $PGF_1$

(422) 7-Benzoylaminothiocarbonyloxy-16-methyl $PGF_1$

(424) 7-Phenylthiocarbonyloxy derivative of (250)

(425) 7-Phenylthiocarbonyloxy derivative of (254)

(426) 7-Phenylthiocarbonyloxy derivative of (258)

(428) 7-Phenylthiocarbonyloxy derivative of (268)

(430) 7-Phenylthiocarbonyloxy derivative of (272)

(432) 7-Phenylthiocarbonyloxy derivative of (280)

(434) 7-Phenoxythiocarbonyloxy derivative of (250)

(436) 7-Phenoxythiocarbonyloxy derivative of (258)

0103445



(438) 7-Phenoxythiocarbonyloxy derivative of (262)
(440) 7-Phenoxythiocarbonyloxy derivative of (276)
(442) 7-Methylthiothiocarbonyloxy derivative of (254)
(444) 7-Methylthiothiocarbonyloxy derivative of (260)
(446) 7-Methylthiothiocarbonyloxy derivative of (268)
(448) 7-Benzoylaminothiocarbonyloxy derivative of (270)
(450) 7-Benzoylaminothiocarbonyloxy derivative of (280)
(452) 7-Imidazothiocarbonyl derivative of (282)
(454) 7-Imidazothiocarbonyl derivative of (286)

As can be seen from the definition of $Z^1$ in formula (II)', the 7-(thiocarboacyloxy)prostaglandins of formula (II)' are structurally characterized by having a thiocarboacyloxy group at the 7-position, and can be produced by starting from the corresponding 7-hydroxyprostaglandins as stated hereinabove.

Investigations of the present inventors have shown that 7-(carboacyloxy)prostaglandins represented by the following formula (III)

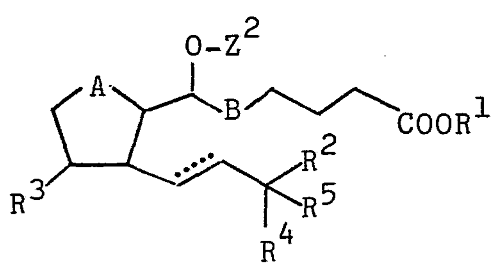 ......... (III)

wherein $R^1$, $R^2$, $R^3$, $R^4$, $R^5$, A and the symbol .... are as defined in formula (II), B is as defined in formula (I), and $Z^2$ represents a carboacyl group,

which have a carboacyloxy group at the 7-position and can be produced from the 7-hydroxyprostaglandins in the same way as in the production of the 7-(thiocarbo-acyloxy)prostaglandins of formula (II)' are as useful as the 7-(thiocarboacyloxy)prostaglandins of formula (II)'.

In formula (III), $R^1$ is preferably a hydrogen atom or a $C_{1-6}$ alkyl group, and $R^2$ is preferably an unsubstituted $C_{1-10}$ alkyl group, a $C_{1-10}$ alkyl group substituted by phenoxy, trifluoromethyl or trifluoromethylphenoxy, an unsubstituted 5- or 6-membered cycloalkyl group, or a 5- or 6-membered cycloalkyl group substituted by $C_{1-6}$ alkyl, trifluoromethyl, phenoxy or trifluoromethylphenoxy. $R^3$ and $R^4$ are indentical or different ana each represents a hydrogen atom, a hydroxyl group, a tri($C_{1-7}$ hydrocarbon)silyl group, or a group capable of forming an acetal linkage with the oxygen atom of the hydroxyl group.

$Z^2$ is a carboacyl group of the following formula

$$-\underset{\overset{\|}{O}}{C}-C_{1-10}\ \text{alkyl}\ .$$

The 7-(carboacyloxy)prostaglandins of formula (II) can be produced in the same way as in the method of producing the 7-(thiocarboacyloxy)prostaglandins E of formula (II)-1 from the 7-hydroxyprostaglandins E of formula (a) and the halogen compounds of formula (b) and the method of producing the 7-(thiocarboacyloxy)-prostaglandins F of formula (II)-2 by reducing the 7-(thiocarboacyloxy)prostaglandins E, by reacting a 7-hydroxy-prostaglandin E of the following formula

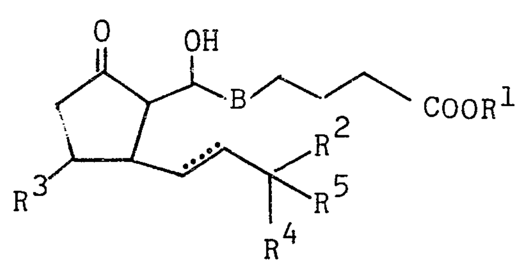

wherein $R^1$, $R^2$, $R^3$, $R^4$, $R^5$, B and the symbol .... are the same as defined hereinabove, with a carboxylic acid halide of the following formula (c)

$$X-Z^2 \quad .................. \quad (c)$$

wherein $Z^2$ is the same as defined in formula (II) and X represents a halogen atom such as a chlorine or bromine atom,

preferably in the presence of a basic compound, and thereafter, as required, subjecting the product to deprotection and/or hydrolysis to form a 7-(carboacyloxy)-prostaglandin E of the following formula (III)-1

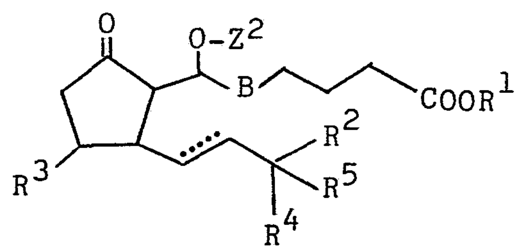

........ (III)-1

wherein $R^1$, $R^2$, $R^3$, $R^4$, $R^5$, B, $Z^2$ and the symbol .... are the same as defined hereinabove,

and subjecting the resulting 7-(carboacyloxy)prostaglandin E to a reaction of converting the carbonyl group at the 9-position to a hydroxymethylene group by reducing it in a manner known *per se*, and thereafter, as required, subjecting the product to deprotection and/or hydrolysis to form a 7-(carboacyloxy)prostaglandin F of the formula (III)-2

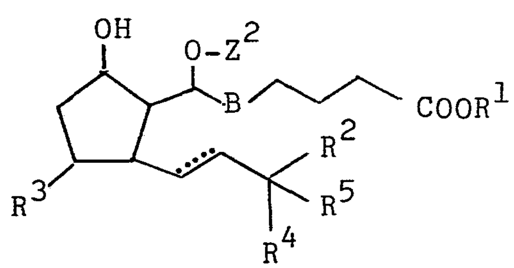

....... (III)-2

wherein $R^1$, $R^2$, $R^3$, $R^4$, $R^5$, B, $Z^2$ and the symbol .... are the same as defined hereinabove.

Examples of the 7-(carboacyloxy)prostaglandins E of formula (III)-1 are given below.

(500) 7-Acetoxy $PGE_1$
(502) 7-Acetoxy-16-methyl $PGE_1$
(504) 7-Acetoxy-16,16-dimethyl-$PGE_1$
(506) 7-Acetoxy-17,20-dimethyl $PGE_1$
(508) 7-Acetoxy-16,17,18,19,20-pentanor-15-cyclohexyl $PGE_1$
(510) 7-Acetoxy-17,17,20-trimethyl $PGE_1$
(512) 7-Acetoxy-18-oxa $PGE_1$
(514) 7-Propionyloxy $PGE_1$
(516) 7-Propionyloxy-15-methyl $PGE_1$
(518) 7-Butyryloxy-16,16-dimethyl $PGE_1$
(520) 7-Butyrloxy-16,17,18,19,20-pentanor-15-cyclohexyl $PGE_1$
(522) 7-Caproyloxy $PGE_1$
(524) 7-Acetoxy derivative of (200)
(526) 7-Acetoxy derivative of (208)
(528) 7-Propionyloxy derivative of (212)
(530) 7-Butyrloxy derivative of (222)
(532) 7-Valeryloxy derivative of (240)
(534) 7-Caproyloxy derivative of (242)

Examples of the 7-(carboacyloxy)prostaglandins F of formula (III)-2 are given below.

(600) 7-Acetoxy $PGF_1$
(602) 7-Acetoxy-16-methyl $PGF_1$
(604) 7-Acetoxy-16,16-dimethyl $PGF_1$
(606) 7-Acetocy-16,17,18,19,20-pentanor-15-cyclopentyl $PGF_1$
(608) 7-Acetoxy-15-methyl $PGF_1$
(610) 7-Acetoxy-17,20-dimethyl-$PGF_1$
(612) 7-Propionyloxy $PGF_1$
(614) 7-Propionyloxy-15-methyl $PGF_1$
(616) 7-Propionyloxy-16,16-dimethyl $PGF_1$
(618) 7-Butyloxy $PGF_1$
(620) 7-Butyloxy-16,17,18,19,20-pentanor-15-cyclohexyl $PGF_1$
(622) 7-Varelyloxy $PGF_1$
(624) 7-Caproyloxy-16,17,18,19,20-pentanor-15-

cyclopentyl $PGF_1$

(626) 7-Acetoxy derivative of (250)
(628) 7-Acetoxy derivative of (254)
(630) 7-Acetoxy derivative of (260)
(632) 7-Acetoxy derivative of (272)
(634) 7-Propionyloxy derivative of (250)
(636) 7-Propionyloxy derivative of (254)
(638) 7-Propionyloxy derivative of (256)
(640) 7-Butyrloxy derivative of (250)
(642) 7-Butyrloxy derivative of (252)
(644) 7-Butyrloxy derivative of (260)
(646) 7-Valeryloxy derivative of (254)
(648) 7-Valeryloxy derivative of (256)
(650) 7-Valeryloxy derivative of (260)
(652) 7-Caproyloxy derivative of (256)
(654) 7-Caproyloxy derivative of (260)
(656) 7-Enanthyloxy derivative of (250)
(658) 7-Enanthyloxy derivative of (280)

The novel 7-(thiocarboacyloxy or carboacyloxy)-prostaglandins provided by this invention are interesting as intermediates for producing natural-type PGE or PGF, or as compounds having useful biological activities such as platelet aggregation inhibiting activity, antiulcer activity, hypotensive activity and labor inducing activity.

The following examples illustrate the present invention in more detail. These examples do not in any way limit the present invention.

Example 1

Synthesis of 7-phenylthiocarbonyloxy-5,6-dehydroprostaglandin $E_2$ methyl ester 11,15-bis(t-butyldimethylsilyl) ether:-

200 mg (0.33 mmole) of 7-hydroxy-5,6-dehydroprostaglandin $E_2$ methyl ester 11,15-bis(t-butyldimethylsilyl)ether was dissolved in 20 ml of dry dichloromethane, and 136 mg (1.11 mmoles) of 4-dimethylaminopyridine was added. At 0 $^{o}C$, 160 mg (1.01 mmoles) of

thiobenzoyl chloride was added by a syringe directly. The temperature of the mixture was raised to 18 °C, and it was stirred for 3 hours. Dichloromethane (20 ml) was added to dilute the reaction mixture, and then it was washed with a 1N dilute aqueous solution of hydrochloric acid, water and a saturated aqueous solution of sodium bicarbonate. The washed product was dried over anhydrous sodium sulfate, and concentrated under reduced pressure. The residue was subjected to silica gel chromatography (10 g, ethyl acetate:hexane:benzene = 1:15:2) to obtain a yellow oil (149.4 mg; 62.4 %; low polarity). From $^1H$ NMR, the main product, 7-phenylthiocarbonyloxy-5,6-dehydroprostaglandin $E_2$ methyl ester 11,15-bis(t-butyldimethylsilyl)ether, was determined to be a mixture of two stereoisomers. This is considered to be due to the steric configuration of C-7 and C-8 (threo and erythro forms).

The spectral data of the product are as follows:-

TLC: Rf = 0.29 (hexane:ethyl acetate = 5:1)

IR (liquid film): 2230, 1743, 1596, 1210 $cm^{-1}$.

$^1H$ NMR($CDCl_3$)δ: 0.1 and 0.17 (each S, 12, S.$CH_3$ x 4), 0.8 - 1.1 (m, 21, C-$CH_3$ x 7), 1.2 - 1.6 (m, 8, $CH_2$ x 4), 1.8 - 3.3 (m, 10, $CH_2$, $CH_2CO$ x 2, $CH_2C\equiv$, and CH x 2), 3.74 (S, 3, $OCH_3$), 3.9 - 4.4 (m, 2, CHOSi x 2), 5.6 - 5.8 (m, 2, vinyl), 6.37 and 6.66 (br, 1, CHOC(=S)), 7.3 - 7.6 (m, 3, phenyl), 8.1 - 8.4 (m, 2, phenyl).

Example 2

Synthesis of 7-phenylthiocarbonyloxy-5,6-dehydroprostaglandin $E_2$ methyl ester 11,15-bis(t-butyldimethylsilyl)ether and its 15-epi-enantiomer:-

126 mg (0.207 mmole) of a mixture of 7-hydroxy-5,6-dehydroprostaglandin $E_2$ methyl ester 11,15-bis(t-butyldimethylsilyl)ether and its 15-epi-enantiomer was dissolved in 2.5 ml of dry methylene chloride, and 55 mg (0.45 mmole) of 4-dimethylaminopyridine was added.

At 21 °C, a solution of 65 mg (0.41 mmole) of thiobenzoyl chloride in 0.5 ml of methylene chloride was added dropwise with stirring. The mixture was stirred at this temperature for 70 minutes, and then diluted with 20 ml of methylene chloride. It was washed with 20 ml of a saturated aqueous solution of sodium bicarbonate, 20 ml of dilute hydrochloric acid and then 20 ml of a saturated aqueous solution of sodium chloride, and dried over anhydrous magnesium sulfate. The dried product was concentrated under reduced pressure, and subjected to silica gel column chromatography (15 g, hexane:ethyl acetate = 10:1) to give a mixture of 7-phenylthiocarbonyloxy-5,6-dehydroprostaglandin $E_2$ methyl ester 11,15-bis-(t-butyldimethylsilyl)ether and its 15-epi-enantiomer as a yellowish brown oil [102.6 mg, 68%; less polar; Rf = 0.29 (hexane:ethyl acetate = 5:1)].

The spectral data of the product were as follows:-

TLC: Rf = 0.29 (hexane:ethyl acetate = 5:1)

IR (neat): 2230 (C≡O), 1748 (C=O), 1212 (C=S) $cm^{-1}$.

$^1H$ NMR($CDC\ell_3$)δ: 0.8 - 1.0 (m, 21, C-$CH_3$ x 7), 1.0 - 1.7 (m, 8, $CH_2$), 1.7 - 2.0 (m, 2, $CH_2$), 2.0 - 3.0 (m, 8, $CH_2CO$ x 2, $CH_2C$≡ and CH x 2), 3.66 (s, 3, $OCH_3$), 3.8 - 4.4 (br, 2, $CHOS_1$ x 2), 5.44 - 5.76 (m, 2, vinyl), 6.30 and 6.57 (br, 1, CHOC(=S)), 7.20 - 7.68 (m, 3, phenyl), 8.04 - 8.34 (m, 2, phenyl).

Anal. Calcd for $C_{40}H_{64}O_6SSi_2$: C, 65.88, H, 8.85;
Found: C, 65.83, H, 8.78.

<u>Examples 3 to 7</u>

The compounds indicated in Table 1 were each synthesized in the same way as in Example 1. Their spectral data are summarized in Table 1.

Table 1

| Example | Compound | Spectral data | | |
|---|---|---|---|---|
| | | NMR [$CDCl_3$, δ(ppm)] | IR (liquid film, $cm^{-1}$) | MASS (20eV, m/e) |
| 3 | 7-Phenylthiocarbonyloxy-5,6-dehydro-16,17,18,19,20-pentanor-15-cyclopentyl $PGE_2$ methyl ester 11,15-bis(t-butyldimethylsilyl)ether | 3.65(s, 3H), 5.5(m, 2H), 6.3 and 6.5(br, 1H), 7.2 - 8.4(m, 5H) | 2230<br>1750<br>1600 | 669($M^+$-t-Bu)<br>558($M^+$-138) |
| 4 | 7-Phenylthiocarbonyloxy-5,6-dehydro-16,17,18,19,20-pentanor-15-cyclohexyl $PGE_2$ methyl ester 11,15-bis(t-butyldimethylsilyl)ether | 3.60(s, 3H), 5.45(m, 2H), 6.35 and 6.6(br. 1H), 7.2 - 8.4(m, 5H) | 2235<br>1750<br>1595 | 683($M^+$-t-Bu)<br>602($M^+$-138) |
| 5 | 7-Phenylthiocarbonyloxy-5,6-dehydro-17(S),20-dimethyl $PGF_2$ methyl ester 11,15-bis(t-butyldimethylsilyl)ether | 3.60(s, 3H), 5.50(m, 2H), 6.3 and 6.7(br, 1H), 7.2 - 8.4(m, 5H) | 2230<br>1745<br>1600 | 699($M^+$-t-Bu)<br>618($M^+$-138) |
| 6 | 7-Phenylthiocarbonyloxy-5,6-dehydro-17(R),20-dimethyl $PGE_2$ methyl ester 11,15-bis(t-butyldimethylsilyl)ether | 3.60(s, 3H), 5.50(m, 2H), 6.3 and 6.7(br, 1H), 7.2 - 8.4(m, 5H) | 2230<br>1745<br>1600 | 699($M^+$-t-Bu)<br>618($M^+$-138) |
| 7 | 7-Phenylthiocarbonyloxy-5,6-dehydro-15-methyl $PGE_2$ methyl ester 11,15-bis(t-butyldimethylsilyl)ether | 1.2(s, 3H), 3.65(s, 3H), 5.5(m, 2H), 6.2 and 6.6(br, 1H), 7.2 - 8.5(m, 5H) | 2235<br>1750<br>1595 | 685($M^+$-t-Bu)<br>604($M^+$-138) |

Example 8

Synthesis of 7-phenylthiocarbonyloxy-5,6-dehydroprostaglandin $F_2$ methyl ester 11,15-bis(t-butyldimethylsilyl)ether:-

7.2 mg (0.01 mmole) of a mixture of 7-phenylthiocarbonyloxy-5,6-dehydroprostaglandin $E_2$ methyl ester 11,15-bis(t-butyldimethylsilyl)ether and its 15-epi-enantiomer was dissolved in 0.5 ml of methanol, and at 0 $^oC$, an excess (4 to 5 mg) of sodium borohydride powder was added by means of a spatula. The mixture was stirred for 19 minutes, and a saturated aqueous sodium of ammonium chloride was added. The reaction mixture was poured into 10 ml of methylene chloride, and 10 ml of a saturated aqueous solution of ammonium chloride was added to extract it. The aqueous layer was further extracted with 10 ml of methylene chloride. The organic layers were combined, dried over anhydrous sodium sulfate, and concentrated under reduced pressure. The residue was subjected to silica gel column chromatography (1 g, ethyl acetate:hexane = 1:5) to give a mixture of 7-phenylthiocarbonyloxy-5,6-dehydroprostaglandin $F_2$ methyl ester 11,15-bis(t-butyldimethylsilyl)ether and its 15-epi-enantiomer [6.9 mg; a 1:1 mixture of $F_{2\alpha}$ and $F_{2\beta}$ forms having an Rf = 0.37 and 0.35 in TLC (ethyl acetate:hexane = 1:5)].

The spectral data of the product are as follows:-

IR (liquid film): 3600 - 3200, 1740, 1595, 1450, 1313, 785 $cm^{-1}$.

$^1H$ NMR($CDCl_3$)$\delta$: 0.05 - 0.10 (m, 12, s, $CH_3 \times 4$), 0.8 - 1.0 (br, s, 21, C-$CH_3 \times 7$), 1.1 - 1.5 (br, 8, $CH_2 \times 4$), 1.58 (s, 1, OH), 2.7 - 3.3 (m, 10, $CH_2CO$, $CH_2 \times 2$, $CH_2C\equiv$, CH x 2), 3.69 (s, 3, $OCH_3$), 4.10 (br, 2, CHO x 2), 4.49 (br, 1, CHO), 5.3 - 5.6 (m, 2, vinyl), 6.34 (d, 0.63H, J=7.5 Hz, CHOC(=S)). 6.60 (d, 0.33H, J=9.0 Hz, CHOC(=S)), 7.45 (m, 3, phenyl), 8.20 (dd, 2, J=9.2 and 2 Hz).

Example 9

Synthesis of 7-phenylthiocarbonyloxy-5,6-dehydroprostaglandin $F_{2\alpha}$ methyl ester 11,15-bis(t-butyldimethylsilyl)ether:-

A solution of diisobutyl aluminum hydride (1 equivalent)/2,6-di-t-butyl-4-methylphenol (2 equivalents) (0.192 M solution, 0.68 ml, 0.14 mmole) was added at -78 °C to a solution of 9.3 mg (0.013 mmole) of a mixture of 7-phenylthiocarbonyloxy-5,6-dehydroprostaglandin $E_2$ methyl ester 11,15-bis(t-butyldimethylsilyl)-ether and its 15-epi-enantiomer in 0.5 ml of toluene in an atomosphere of argon. The mixture was stirred at -20 °C for 25 minutes. Then, 5 ml of a saturated aqueous solution of sodium hydrogen tartrate was added, and the mixture was extracted with 20 ml of ethyl acetate. The extract was dried over anhydrous sodium sulfate, and concentrated under reduced pressure. The product showed two spots (about 1:1, Rf = 0.32 and Rf = 0.2 (ethyl acetate:hexane = 1:5) on its thin-layer chromatogram. The product was subjected to silica gel column chromatography (2 g, ethyl acetate:hexane = 1:5) to give 8.5 mg of a reduction product, i.e. a mixture of 7-phenylthiocarbonyloxy-5,6-dehydroprostaglandin $F_{2\alpha}$ methyl ester 11,15-bis(t-butyldimethylsilyl)ether and its 15-epi-enantiomer.

Examples 10 to 14

The compounds indicated in Table 2 were synthesized in the same way as in Example 9. Their spectral data are summarized in Table 2.

Table 2

| Example | Compound | Spectral data | | |
|---|---|---|---|---|
| | | NMR [$CDCl_3$, $\delta$(ppm)] | IR (liquid film, $cm^{-1}$) | MASS (20eV, m/e) |
| 10 | 7-Phenylthiocarbonyloxy-5,6-dehydro-16,17,18,19,20-pentanor-15-cyclopentyl $PGF_{2\alpha}$ methyl ester 11,15-bis(t-butyldimethylsilyl)ether | 3.65 (s, 3H)<br>5.5 (m, 2H)<br>6.3 and 6.5 (br, 1H)<br>7.2 - 8.4 (m, 5H) | 3350<br>1740<br>1600 | 671 ($M^+$-t-Bu)<br>710 ($M^+$-$H_2O$) |
| 11 | 7-Phenylthiocarbonyloxy-5,6-dehydro-16,17,18,19,20-pentanor-15-cyclohexyl $PGF_{2\alpha}$ methyl ester 11,15-bis(t-butyldimethylsilyl)ether | 3.60 (s, 3H)<br>5.45 (m, 2H)<br>6.35 and 6.6 (br, 1H)<br>7.2 - 8.4 (m, 5H) | 3450<br>1745<br>1600 | 685 ($M^+$-t-Bu)<br>724 ($M^+$-$H_2O$) |
| 12 | 7-Phenylthiocarbonyloxy-5,6-dehydro-17(S),20-dimethyl $PGF_{2\alpha}$ methyl ester 11,15-bis(t-butyldimethylsilyl)ether | 3.60 (s, 3H)<br>5.50 (m, 2H)<br>6.3 and 6.7 (br, 1H)<br>7.2 - 8.4 (m, 5H) | 3450<br>1740<br>1595 | 701 ($M^+$-t-Bu)<br>740 ($M^+$-$H_2O$) |
| 13 | 7-Phenylthiocarbonyloxy-5,6-dehydro-17(R),20-dimethyl $PGF_{2\alpha}$ methyl ester 11,15-bis(t-butyldimethylsilyl)ether | 3.60 (s, 3H)<br>5.50 (m, 2H)<br>6.3 and 6.7 (br, 1H)<br>7.2 - 8.4 (m, 5H) | 3350<br>1745<br>1595 | 701 ($M^+$-t-Bu)<br>740 ($M^+$-$H_2O$) |
| 14 | 7-Phenylthiocarbonyloxy-5,6-dehydro-15-methyl $PGF_{2\alpha}$ methyl ester 11,15-bis(t-butyldimethylsilyl)ether | 1.2 (s, 3H), 3.65 (s, 3H)<br>5.5 (m, 2H)<br>6.2 and 6.6 (br, 1H)<br>7.2 - 8.5 (m, 5H) | 3500<br>1745<br>1595 | 687 ($M^+$-t-Bu)<br>726 ($M^+$-$H_2O$) |

Example 15

Synthesis of 7-acetoxy-5,6-dehydroprostaglandin $E_2$ methyl ester 11,15-bis(t-butyldimethylsilyl) ether:-

100 mg (0.165 mmole) of 7-hydroxy-5,6-dehydroprostaglandin $E_2$ methyl ester 11,15-bis(t-butyldimethylsilyl)ether was dissolved in 1 ml of anhydrous pyridine and 1 ml of carbon tetrachloride, and with stirring under ice cooling, 15 mg (0.20 mmole) of acetyl chloride was slowly added. The reaction was performed for 1 hour. The reaction product was worked up in a customary manner to give about 93 mg of an oily product identified as 7-acetoxy-5,6-dehydroprostaglandin $E_2$ methyl ester 11,15-bis(t-butyldimethylsilyl)ether.

The spectral data of the product are as follows:-

TLC: Rf = 0.55 (ethyl acetate:benzene = 10:90)

IR (liquid film, $cm^{-1}$): 2230, 1750, 1600 $cm^{-1}$.

NMR ($CDCl_3$, δ(ppm)): 2.05 (s, 3H),
3.65 (s, 3H),
5.45 (m, 2H).

Example 16

Synthesis of 3-butyl-2-(6-carbomethoxy-1-phenylthiocarbonyloxy-2-hexynyl)cyclopentanone:-

532 mg (1.81 mmoles) of 3-butyl-2-(1-hydroxy-6-carbomethoxy-2-hexynyl)cyclopentanone was dissolved in 6 ml of dry dichloromethane, and 423 mg (2.70 mmoles) of 4-dimethylaminopyridine was added. The mixture was cooled to 10 °C. A solution of 423 mg (2.70 mmoles) of thiobenzoyl chloride in 1.0 ml of dry dichloromethane was added dropwise by means of a syringe. Furthermore, 0.5 ml of dry dichloromethane was added by the syringe in a manner such that it washed with inside of the syringe. The mixture was stirred at 10 to 16 °C for 50 minutes, and then diluted with 20 ml of dichloromethane. The diluted mixture was washed with 20 ml of a saturated aqueous solution of sodium bicarbonate, 20 ml of 1N hydrochloric acid and 20 ml of a saturated aqueous solution of sodium chloride, dried over anhydrous magnesium sulfate, and

concentrated. The residue was subjected to silica gel column chromatography (30 g, hexane:ethyl acetate = 6:1) to give 722 mg (96 %) of 3-butyl-2-(6-carbomethoxy-1-thiobenzoyloxy-2-hexynyl)cyclopentanone.

The spectral data of the product (a mixture of two isomers) are as follows:

TLC: Rf = 0.34 (hexane:ethyl acetate = 3:1)

IR(neat, $cm^{-1}$): 2238 (C ≡ C), 1746 (C=O), 1599(phenyl), 7206 (C=S), 771(phenyl), 685(phenyl).

$^1H$ NMR($CDCl_3$, 90 MHz, ppm) δ: 8.2 - 8.0 (m, 2H, O-), 7.6 - 7.2 (m, 3H, m-, p-), 6.64 (m, 0.33H, -CH-O-), 6.42 (m, 0.67H, -CH-O-), 3.64 (s, 3H, $-OCH_3$), 2.7 - 2.1 (m, 8H, -CH-, $-CH_2-C=O$, $-CH_2-C≡C-$), 2.1 - 1.1 (m, $-CH_2-$), 0.94 (t, 3H, $-CH_2CH_3$).

MS (m/e, 75eV): 414 ($M^+$).

Example 17

Synthesis of 3-butyl-2-(6-carbomethoxy-1-phenoxythiocarbonyloxy-2-hexynyl)-cyclopentanone:-

300 mg (1.0 mmole) of 3-butyl-2-(6-carbomethoxy-1-hydroxy-2-hexynyl)cyclopentanone was dissolved in 5 ml of dry dichloromethane, and 280 mg (1.80 mmoles) of 4-dimethylaminopyridine was added. The mixture was cooled to 0 °C. A solution of 264 mg (1.70 mmoles) of phenoxythiocarbonyl chloride in 0.5 ml of dichloromethane was added, and the mixture was stirred at 0 to 16 °C for about 30 minutes. After the reaction, the reaction mixture was worked up in a customary manner, and subjected to silica gel column chromatography (30 g, hexane:ethyl acetate = 6:1) to give 315 mg of 3-butyl-2-(6-carbomethoxy-1-phenoxythiocarbonyloxy-2-hexynyl)-cyclopentanone.

The spectral data of the product are as follows:-

TLC: Rf = 0.40 (hexane:ethyl acetate = 3:1)

IR(neat, $cm^{-1}$): 2240, 1750, 1600

NMR($CDCl_3$, δ(ppm)): 8.3 - 7.2 (5H, m),

BAD ORIGINAL

6.6 and 6.45 (m, 1H),
3.65 (s, 3H).

Example 18

Synthesis of 5,6-dehydroprostaglandin $E_2$ methyl ester 11,15-bis(t-butyldimethylsilyl) ether:-

233.4 mg (0.32 mmole) of 7-phenylthiocarbonyloxy-5,6-dehydroprostaglandin $E_2$ methyl ester 11,15-bis(t-butyldimethylsilyl)ether was dissolved in 2 ml of tributyltin hydride in an atomosphere of argon, and 8 mg of bis-t-butyl peroxide was added. The mixture was stirred at 50 °C for 35 minutes. The reaction mixture was cooled to room temperature and directly chromatographed on a silica gel column (20 g of silica gel; ethyl acetate: hexane:benzene = 1:15:2) to give 157.4 mg (83 %) of 5,6-dehydroprostaglandin $E_2$ methyl ester 11,15-bis(t-butyldimethylsilyl) ether as a colorless oil.

The spectral data of the resulting product are shown below.

TLC: Rf = 0.50 (ethyl acetate:hexane = 1:5)

IR (neat): 1746, 1246, 827, 767 $cm^{-1}$.

$^1H$ NMR ($CDCl_3$:$CCl_4$ = 1:1) δ:

0.04 and 0.06 (each s, 12, $SiCH_3$ x 2),
0.89 (s, 18, $SiC(CH_3)_3$ x 2),
0.92 (t, 1, J=6.5 Hz, $CH_3$),
1.1 - 1.5 (m, 8, $CH_2$ x 4),
1.7 - 2.9 (m, 12, $CH_2CO$ x 2, $CH_2C\equiv$ x 2, CH x 2, and $CH_2$),
3.65 (s, 3, $OCH_3$), 4.05 (m, 2, CHOSi x 2),
5.4 - 5.7 (m, 2, vinyl).

$^{13}C$ NMR ($DCDl_3$) δ:

-4.7, -4.5 (for two), -4.2, 13.6, 14.0, 16.9, 18.0, 18.2, 22.6, 24.2, 25.0, 25.8 (for three), 25.9 (for three), 31.9, 32.7, 38.6, 47.7, 51.4, 51.9, 52.9, 72.7, 73.1, 77.3, 80.8, 128.2, 136.8, 173.4, 213.4.

$[\alpha]_D^{21}$: $-13.9^o$ (C = 1.59, $CH_3OH$)

Examples 19 to 22

By the same procedure as in Example 18, PGE-type compounds were obtained from the corresponding 7-phenylthiocarbonyloxy compound as shown in Table 3. The spectral data of the products are summarized in Table 3.

Table 3

| Example | Compound formed | Spectral data | | |
| --- | --- | --- | --- | --- |
| | | IR (neat, $cm^{-1}$) | NMR ($CDCl_3$, $\delta$(ppm)) | Mass (20 eV, m/e) |
| 19 | 5,6-Dehydro-15-methyl $PGE_2$ methyl ester 11,15-bis(t-butyldimethylsilyl) ether | 1745 | 1.23 (s, 3H),<br>3.65 (s, 3H),<br>5.45 (m, 2H). | 549 ($M^+$-t-Bu) |
| 20 | 5,6-Dehydro-17(S),20-dimethyl $PGE_2$ methyl ester 11,15 - bis(t-butyldimethyl-silyl) ether | 1743 | 1.05 (d, J=7Hz, 3H),<br>3.65 (s, 3H),<br>5.50 (m, 2H). | 563 ($M^+$-t-Bu) |
| 21 | 5,6-Dehydro-16,17,18,19,20-pentanor-15-cyclohexyl $PGE_2$ methyl ester 11,15-bis(t-butyldimethylsilyl) ether | 1745 | 1.1 - 1.5 (10H),<br>3.65 (s, 3H),<br>5.50 (m, 2H). | 547 ($M^+$-t-Bu) |
| 22 | 5,6-Dehydro-16,17,18,19,20-pentanor-15-cyclopentyl $PGE_2$ methyl ester 11,15-bis(t-butyldimethylsilyl) ether | 1745 | 1.1 - 1.5 (8H),<br>3.65 (s, 3H),<br>5.45 (m, 2H). | 533 ($M^+$-t-Bu) |

Example 23

Synthesis of 5,6-dehydroprostaglandin $F_2$ methyl ester 11,15-bis(t-butyldimethylsilyl) ether:-

(i) 7.2 mg (0.10 mmole) of 7-phenylthiocarbonyloxy-5,6-dehydroprostaglandin $E_2$ methyl ester 11,15-bis(t-butyldimethylsilyl) ether was dissolved in 0.5 ml of methanol, and while the solution was stirred at 0 $^{o}C$, an excess of (4 to 5 mg) $NaBH_4$ (powder) was added by means of a spatula. After the mixture was stirred for 10 minutes, a saturated aqueous solution of $NH_4Cl$ was added. The reaction mixture was poured into 10 ml of dichloromethane, and 10 ml of a saturated aqueous solution of $NH_4Cl$ was added. Thus, the reaction mixture was extracted. The aqueous layer was further extracted with 10 ml of $CH_2Cl$. The organic layers were combined and dried over $Na_2SO_4$. The dried product was concentrated under reduced pressure, and subjected to silica gel chromatography (1 g, ethyl acetate-hexane = 1:5) to give a mixture of 7-phenylthiocarbonyloxy-5,6-dehydroprostaglandin $F_{2\alpha}$ methyl ester 11,15-bis(t-butyldimethylsilyl) ether and 7-phenylthiocarbonyloxy-5,6-dehydroprostaglandin $F_{2\beta}$ methyl ester 11,15-bis(t-butyldimethylsilyl) ether [6.9 mg; Rf: 0.37 and 0.35 (ethyl acetate:hexane = 1:5)].

IR (liquid film): 3600 - 3200, 1740, 1595, 1450, 1313, 785 $cm^{-1}$.

$^1H$ NMR ($CDCl_3$) $\delta$:

0.05 - 0.10 (m, 12, s, $CH_3 \times 4$),
0.8 - 1.0 (brs, 21, C-$CH_3 \times 7$),
1.1 - 1.5 (br, 8, $CH_2 \times 4$), 1.58 (s, 1, OH),
2.7 - 3.3 (m, 10, $CH_2CO$, $CH_2 \times 2$, $CH_2C\equiv$, CH x 2),
3.69 (s, 3, $OCH_3$),
4.10 (br, 2, CHO x 2), 4.49 (br, 1, CHO),
5.3 - 5.6 (m, 2, vinyl),
6.34 (d, 0.63H, J=7.5 Hz, CHOC(=S)),
6.60 (d, 0.33H, J=9.0 Hz, CHOC(=S)),
7.45 (m, 3, phenyl),
8.20 (dd, 2, J=9.2 and 2 Hz).

(ii) The mixture obtained in (i) above (6.9 mg) was dissolved in 0.1 ml of tributyltin hydride, and 4 mg of bis-t-butyl peroxide was added. The mixture was heated at 27 °C for 10 minutes and then at 50 °C for 15 minutes. The desired alcohol compound was obtained on TLC (ethyl acetate-hexane = 1:5).

The reaction mixture was directly subjected to silica gel column chromatography (3 g, ethyl acetate: hexane = 1:10 → 1:5) to give 5.8 mg of 5,6-dehydroprostaglandin $F_2$ methyl ester 11,15-bis(t-butyldimethylsilyl) ether. Since this product contained some tri-n-butyltin hydride, it was again subjected to silica gel column chromatography (1.5 g; ethyl acetate:hexane:benzene = 1:7.5:1) to give 5,6-dehydroprostaglandin $F_2$ methyl ester 11,15-bis(t-butyldimethylsilyl) ether (4.1 mg; yield from 5,6-dehydroprostaglandin $E_2$ methyl ester 11,15-bis(t-butyldimethylsilyl) ether, 73 %).

The spectral data of 5,6-dehydroprostaglandin $F_2$ methyl ester 11,15-bis(t-butyldimethylsilyl) ether were as follows:-

TLC: Rf = 0.29 (ethyl acetate:hexane = 1:5).

IR (liquid film):

3640 - 3080, 1745, 1247, 1020, 970, 930, 830, 770 $cm^{-1}$.

$^1$H NMR ($CDCl_3$) δ:

0.02 and 0.05 (each 12, $SiCH_3$ x 4),
0.7 - 1.0 (m, 21, C-$CH_3$ x 7),
2.1 - 3.5 (m, 20, $CH_2CO$, $CH_2$ x 6, $CH_2C\equiv$ x 2, CH x 2), 3.69 (d, 1, J=8.3 Hz, OH),
3.67 (s, 3, $OCH_3$),
4.00 and 4.24 (br, 3, CHO x 3),
5.40 (m, 2, vinyl)

$[\alpha]_D^{21}$: +0.37° (C = 0.715, $CH_3OH$)

Examples 24 to 27

In the same way as in Example 23, PGF-type compounds were obtained from the corresponding 7-phenylthiocarbonyloxy PGE compounds as shown in Table 4. The spectral data of the products are summarized in Table 4.

Table 4

| Example | Compound produced | Spectral data | | |
|---|---|---|---|---|
| | | IR (neat, $cm^{-1}$) | NMR ($CDCl_3$, δ(ppm)) | Mass (20 eV, m/e) |
| 24 | 5,6-Dehydro-15-methyl $PGF_{2\alpha}$ methyl ester 11,15-bis(t-butyldimethylsilyl) ether | 3500, 1745 | 1.21 (s, 3H), 3.65 (s, 3H), 4.02 (m, 2H), 5.45 (m, 2H). | 590 ($M^+$-$H_2O$), 551 ($M^+$-t-Bu). |
| 25 | 5,6-Dehydro-17,20-dimethyl $PGE_{2\alpha}$ methyl ester 11,15-bis(t-butyldimethylsilyl) ether | 3350, 1745. | 0.95 (d, J=7Hz, 3H), 3.65 (s, 3H), 4.03 (m, 2H), 5.50 (m, 2H). | 604 ($M^+$-$H_2O$), 565 ($M^+$-t-Bu). |
| 26 | 5,6-Dehydro-16,17,18,19,20-pentanor-15-cyclohexyl $PGF_{2\alpha}$ methyl ester 11,15-bis(t-butyldimethylsilyl) ether | 3350, 1745. | 1.1 - 1.5 (10H), 3.60 (s, 3H), 4.05 (m, 2H), 5.50 (m, 2H). | 588 ($M^+$-$H_2O$), 549 ($M^+$-t-Bu). |
| 27 | 5,6-Dehydro-16,17,18,19,20-pentanor-15-cyclopentyl $PGF_{2\alpha}$ methyl ester 11,15-bis(t-butyldimethylsilyl) ether | 3330, 1745 | 1.1 - 1.5 (8H), 3.65 (s, 3H), 4.10 (m, 2H), 5.50 (m, 2H). | 574 ($M^+$-$H_2O$), 535 ($M^+$-t-Bu). |

Example 28

Synthesis of 3-butyl-2-(6-carbomethoxy-2-hexynyl)cyclopentanone:-

21.4 mg (51.6 μmoles) of 3-butyl-2-(6-carbomethoxy-1-thiobenzoyloxy-2-hexynyl)cyclopentanone and 0.5 ml (3 μmoles) of α,α-azobisisobutyronitrile (AIBN) were dissolved in 1.0 ml of dry tetrahydrofuran (THF), and the solution was heated to 50 °C. A solution of 30.0 mg (103 μmoles) of tributyltin hydride in 0.5 ml of dry THF was added dropwise to the heated solution over 2 minutes. The mixture was stirred for 9 hours at this temperature. Then, the mixture was concentrated, and subjected to silica gel column chromatography (3 g of Merck 7734 silica gel treated with 6 % water; hexane: ethyl acetate = 6:1) to give 11.4 mg (79 %) of 3-butyl-2-(6-carbomethoxy-2-hexynyl)cyclopentanone.

The spectral data of the product are as follows:

Silica gel TLC: Rf ≐ 0.41 (hexane:ethyl acetate = 3:1)

IR (neat, $cm^{-1}$): 1747 (C=O).

$^1H$ NMR ($CDCl_3$, 90 MHz, ppm) δ:

3.66 (s, 3H, $-OCH_3$),
2.57 - 2.00 (m, 9H, $-CH_2-\dot{C}=O$, $-CH_2-C{\equiv}C-$, $-\dot{C}H-\dot{C}=O$), 1.93 - 1.10 (m, 11H, $-CH_2-$, $-\dot{C}H-$),
0.92 (t, 3H, $-CH_2CH_3$).

$^{13}C$ NMR ($CDCl_3$, 22.5 MHz, ppm) δ:

218.6 (ketone), 173.5 (ester), 80.4 (C≡C), 78.0 (C≡C), 53.9, 51.4, 41.0, 38.0, 34.3, 32.8, 29.3, 27.1, 24.3, 22.9, 18.2, 17.6, 14.0.

Examples 29 to 37

The same conversion reaction as in Example 28 was carried out under various conditions. The results are shown in Table 5.

Table 5

| Example | Solvent | AIBN (mole %) | Temp. (°C) | Time (hours) | Yield (%) |
|---|---|---|---|---|---|
| 29a | Benzene | 5 | 40-50 | 12 | 56 |
| 30a | Benzene | 5 | 75 | 10 | 60 |
| 31a | Toluene | 5 | 75 | 10 | 61 |
| 32a | THF | 5 | 50 | 10 | 74 |
| 33a | Benzene | 2 | 75 | 6 | 66 |
| 34b | THF | 4 | 40 | 3 | 72 |
| 35b | THF | 5 | 60 | 0.5 | 61 |
| 36a | THF | 6 | 50 | 9 | 79 |
| 37a | THF | 6 | 60 | 4 | 74 |

a: The reaction was carried out by dissolving the starting compound and AIBN in the solvent, heating the solution to the reaction temperature, and then adding a solution of tri-n-butyltin hydride in the same solvent as the reaction solvent.

b: The reaction was carried out by mixing the starting material, AIBN and tri-n-butyltin hydride at room temperature and then heating the mixture to the reaction temperature.

Example 38

Synthesis of prostaglandin $E_2$ methyl ester 11,15-bis(t-butyldimethylsilyl) ether:-

48.2 mg (0.081 mmole) of 5,6-dehydroprostaglandin $E_2$ methyl ester 11,15-bis(t-butyldimethylsilyl) ether and 25 mg of synthetic quinoline were dissolved in 2.5 ml of cyclohexane, and subsequently, 25 mg of 5 % Pd-$BASO_4$ was added. The mixture was stirred at 25 °C for 3 hours in an atmosphere of hydrogen, and 50 mg of synthetic quinone and 50 mg of 5 % Pd-$BaSO_4$ were further added. The mixture was stirred at 40 °C for 4.5 hours. The catalyst was removed by filtration, and the filtrate was washed with ethyl acetate and concentrated under reduced pressure.

The concentrate was subjected to silica gel

column chromatography (8 g; diethyl ether:hexane = 1:10). The collected fractions were concentrated under reduced pressure, and the residue was left to stand for 7 hours under reduced pressure (<4 mmHg) produced by a vacuum pump) to give 41.8 mg (87 %) of prostaglandin $E_2$ methyl ester 11,15-bis(t-butyldimethylsilyl) ether.

The spectral data of the resulting product are as follows:-

TLC: Rf = 0.58 (ethyl acetate:hexane = 1:5).

IR (neat): 1743, 1243, 1000, 964, 927, 828, 768 $cm^{-1}$

$^1H$ NMR ($CDCl_3$) $\delta$:

0.03 and 0.06 (each s, 12, $SiCH_3 \times 4$),
0.8 - 1.0 (m, 21, $C-CH_3 \times 7$),
1.2 - 1.5 (m, 8, $CH_2 \times 4$), 1.6 - 2.9 (m, 12, $CH_2CO \times 2$, $CH_2C= \times 2$, and $CH_2$),
3.67 (s, 3, $OCH_3$), 4.06 (m, 2, $CHOSi \times 2$),
5.37 (m, 1, vinyl), 5.54 (m, 1, vinyl).

$[\alpha]_D^{21}$: $-52.7^o$ (C=1.28, $CH_3OH$)

Example 39

Synthesis of prostaglandin $E_2$ methyl ester:-

40 mg (0.067 mmole) of prostaglandin $E_2$ methyl ester 11,15-bis(t-butyldimethylsilyl) ether was dissolved in 8 ml of anhydrous acetonitrile, and at 0 $^oC$, 0.1 ml of HF-pyridine was added. The mixture was stirred at 24 $^oC$ for 30 minutes. The reaction mixture was poured into 20 ml of a saturated aqueous solution of sodium bicarbonate, and extracted with three 30 ml portions of ethyl acetate. The organic layers were combined, dried over anhydrous sodium sulfate, and concentrated under reduced pressure. To remove pyridine from the residue, toluene was added, and the mixture was further concentrated under reduced pressure. The residue was allowed to stand for a while under reduced pressure ( <4 mmHg) produced by a vacuum pump, and then subjected to silica gel column chromatography (2 g; ethyl acetate:hexane = 1:1 → 1:0 gradient) to give 24.1 mg (98 %) of (-)-$PGE_2$ methyl ester.

The spectral data of the resulting product are as follows:-

TLC: Rf = 0.29 (ethyl acetate:cyclohexane:THF = 6:3:1).

IR (liquid film): 3680 - 3080, 1744, 970 $cm^{-1}$.

$^1H$ NMR ($CDCl_3$) δ:

0.90 (t, 1, J=6.5 Hz, $CH_3$), 1.1 - 2.9 (m, 20, $CH_2CO \times 2$, $CH_2 \times 5$, $CH_2C= \times 2$, $CH \times 2$), 3.08 (br, 1, OH), 3.66 (s, 3, $OCH_3$), 4.06 (m, 3, $CHO \times 2$ and OH), 5.34 (m, 1, vinyl), 5.70 (m, 1. vinyl).

$^{13}C$ NMR ($DCDl_3$) δ:

14.0, 22.6, 24.7, 25.1, 26.6, 31.7, 33.5, 37.3, 46.1, 51.5, 53.7, 54.5, 72.0, 73.0, 126.6, 130.8, 131.5, 136.8, 174.0, 214.1.

$[\alpha]_D^{22}$: $-71.7^o$ (C=1.043, $CH_3OH$)

Example 40

Synthesis of 5,6-dehydroprostaglandin $F_{2\alpha}$ methyl ester 11,15-bis(t-butyldimethylsilyl) ether:-

A solution of 25.5 mg (0.043 mmole) of 5,6-dehydroprostaglandin $E_2$ methyl ester 11,15-bis(t-butyldimethylsilyl) ether in 1 ml of toluene was added to a toluene solution (0.19 M, 2.24 ml, 0.43 mmole) of diisobutyl aluminum hydride (1 equivalent)/2,6-di-t-butyl-4-methylpenol (2 equivalents). The mixture was stirred at -78 °C for 2 hours. Then, the temperature was raised, and the mixture was stirred at -25 to -20 °C for 3 hours. 10 ml of a saturated aqueous solution of sodium tartrate was added, and the mixture was vigorously shaken.

The reaction mixture was extracted three times with ethyl acetate (20 + 10 + 10 ml) at room temperature. The organic layers were combined, dried over anhydrous sodium sulfate, and concentrated under reduced pressure.

The residue was subjected to silica gel column chromatography (5 g; ethyl acetate:hexane = 5:1) to obtain 23.5 mg (92 %, less polar component) of

5,6-dehydroprostaglandin $F_{2\alpha}$ methyl ester 11,15-bis(t-butyldimethylsilyl) ether.

The spectral data of the product are as follows:-

TLC: Rf = 0.29 (ethyl acetate:hexane = 1:5).

IR (liquid film): 3640 - 3080, 1745, 1247, 1020, 970, 930, 830, 770 $cm^{-1}$.

$^1H$ NMR ($CDCl_3$) δ:

0.02 and 0.05 (each s, 12, $SiCH_3 \times 4$), 0.7 - 1.0 (m, 21, C-$CH_3 \times 7$), 2.1 - 3.5 (m, 20, $CH_2CO$, $CH_2 \times 6$, $CH_2C\equiv \times 2$, CH x 2), 3.69 (d, 1, J=8.3 Hz, OH), 3.67 (s, 3, $OCH_3$), 4.00 and 4.24 (br, 3, CHO x 3), 5.40 (m, 2, vinyl).

$[\alpha]_D^{21}$: +0.37° (C=0.715, $CH_3OH$)

<u>Example 41</u>

Synthesis of prostaglandin $F_{2\alpha}$ methyl ester 11,15-bis(t-butyldimethylsilyl) ether:-

28.7 mg (0.04 mmole) of 5,6-dehydroprostaglandin $F_{2\alpha}$ methyl ester 11,15-bis(t-butyldimethylsilyl) ether was dissolved in 1 ml of benzene, and 1 ml of cyclohexane and 28.7 mg of a Lindler's catalyst were added. The mixture was stirred at 22 to 23.5 °C for 12 hours in an atmosphere of hydrogen. The catalyst was removed by filtration. The filtrate was washed with ethyl acetate and concentrated under reduced pressure.

The residue was subjected to silica gel column chromatography (6 g; ethyl acetate:hexane:benzene = 1:15:2) to give 23.2 mg (81 %) of prostaglandin $F_{2\alpha}$ methyl ester 11,15-bis(t-butyldimethylsilyl) ether.

The spectral data of this product are as follows:-

TLC: Rf = 0.32 (ethyl acetate:hexane = 1:5)

IR (neat): 3610 - 3280, 1745, 1250, 1000, 970, 938, 830, 770 $cm^{-1}$.

$^1H$ NMR ($CDCl_3$) δ:

0.03 and 0.05 (each s, 12, $SiCH_3 \times 4$), 0.8 - 1.0 (m, 21, C-$CH_3 \times 7$), 1.2 - 2.4 (m, 20, $CH_2CO$, $CH_2 \times 6$, $CH_2C= \times 2$, CH x 2), 2.69 (d, 1, J=9.5 Hz, OH),

3.67 (s, 3, $OCH_3$), 4.05 (br, 3, CHO x 3), 5.40 (m, 2, vinyl).

$[\alpha]_D^{23}$: +12.3° (C=1.037, $CH_3OH$)

The IR and $^1H$ NMR spectra of the above product completely agreed with those of the disilyl compound derived from authentic (+)-$PGF_{2\alpha}$ methyl ester.

Example 42

Synthesis of prostaglandin $F_{2\alpha}$ methyl ester:-

21 mg (0.035 mmole) of prostaglandin $F_{2\alpha}$ methyl ester 11,15-bis(t-butyldimethylsilyl)ether was dissolved in 1 ml of acetic acid, and 0.33 ml of water and 0.1 ml of tetrahydrofuran were added. The mixture was stirred at 55 °C for 1.5 hours, and transferred to a large vessel. Toluene was added, and the reaction mixture was concentrated under reduced pressure several times respectedly to distill off acetic acid and water. The residue was subjected to silica gel column chromatography [3 g; ethyl acetate:hexane = (1:1) → (1:0)] to give 11 mg (85 %) of (+)-$PGF_{\alpha}$ methyl ester.

The spectral data of the product are as follows:

TLC: Rf = 0.2 (ethyl acetate:cyclohexane:tetrahydrofuran = 6:3:1)

IR (liquid film): 3640 - 3040, 1738, 1435, 1160, 1116, 1042, 1020, 968, 858 $cm^{-1}$.

$^1H$ NMR ($CDCl_3$) δ:

0.89 (t, 3, J=6.5 Hz, $CH_3$), 1.2 - 2.4 (m, 20, $CH_2CO$, $CH_2$ x 6, $CH_2C$= x 2, CH x 2), 2.57 (br, 1, OH), 3.2 q (br, 1, OH), 3.69 (s, 3, $OCH_3$), 4.03 (brm, 3, CHO x 3), 5.3 - 5.6 (m, 2, vinyl).

$^{13}C$ NMR ($CDCl_3$) δ:

14.0, 22.6, 24.8, 25.2, 25.6, 26.6, 31.8, 33.5, 37.3, 43.0, 50.5, 51.6, 55.8, 72.9, 73.0 78.0, 129.1, 129.6, 132.6, 135.3, 174.3.

$[\alpha]_D^{20}$: +31.4° (C=0.423, $CH_3OH$)

The spectra (IR, $^1H$ NMR, $^{13}C$ NMR, and TLC) of the above product completely agreed with those of

(+)-$PGF_{2\alpha}$ methyl ester derived from authentic (+)-$PGF_{2\alpha}$.

Example 43

Synthesis of prostaglandin $F_{2\alpha}$ methyl ester 11,15-bis(t-butyldimethylsilyl)ether:-

A toluene solution of diisobutyl aluminum hydride (1 equivalent)/2,6-di-t-butyl-4-methylphenol (2 equivalents) (0.192M solutioh; 2.3 ml; 0.44 mmole) was added with stirring to a solution of 20 mg (0.034 mmole) of prostaglandin $E_2$ methyl ester 11,15-bis(t-butyldimethylsilyl)ether in 1 ml of toluene.

The temperature was raised, and the mixture was stirred at -25 to -20 °C for 2 hours. Then, 10 ml of a saturated aqueous solution of sodium hydrogen tartrate was added, and the mixture was vigorously shaken. It was then extracted three times with ethyl acetate (20 + 10 + 10 ml). The extracts were combined, dried over anhydrous sodium sulfate, and concentrated under reduced pressure. The residue was subjected to silica gel column chromatography (5 g; ether:hexane = 1:5) to give 17.5 mg (87 %) of prostaglandin $F_{2\alpha}$ methyl ester 11,15-bis(t-butyldimethylsilyl) ether (less polar component).

Example 44

Synthesis of prostaglandin $E_1$ methyl ester 11,15-bis(t-butyldimethylsilyl)ether:-

8.1 mg of 5,6-dihydroprostaglandin $E_1$ methyl ester 11,15-bis(t-butyldimethylsilyl)ether was dissolved in 1 ml of methanol, and 5 mg of 5 % Pd-C was added. The mixture was stirred at 0 °C for 12.5 hours in an atmosphere of hydrogen. The catalyst was removed by filtration, and the filtrate was washed with ethyl acetate and concentrated under reduced pressure. The residue was subjected to silica gel column chromatography (3 g; ethyl acetate:hexane = 1:10) to obtain 5.8 mg (71 %) of prostaglandin $E_1$ methyl ester 11,15-bis(t-butyldimethylsilyl)ether.

The spectral data of the resulting product are as follows:-

IR (neat): 1743, 1000, 970, 830, 770 $cm^{-1}$.
$^1H$ NMR ($CDCl_3$) $\delta$: 0.02 and 0.05 (each s, 12, $SiCH_3 \times 4$), 0.88 and 0.90 (each s, 18, $Si((CH_3)_3 \times 2)$, 0.85 - 0.95 (hidden in this region, 3, $CH_3$), 1.05 - 2.8 (m, 24, $CH_2 \times 11$, $CH \times 2$), 3.66 (s, 3, $OCH_3$), 4.08 (m, 2, $CHOSi \times 2$), 5.54 (m, 2, vinyl).
$[\alpha]_D^{21}$: -41.0, (C=0.78, $CH_3OH$).

Example 45

Synthesis of prostaglandin $E_1$ methyl ester:-

4.8 mg (0.008 mmole) of prostaglandin $E_1$ methyl ester 11,15-bis(t-butyldimethylsilyl)ether was dissolved in 0.5 ml of acetonitrile, and at 0 °C, 0.05 ml of HF-pyridine was added. The mixture was stirred at 19 °C for 4 hours. The reaction mixture was poured into 5 ml of a saturated aqueous solution of sodium bicarbonate, and extracted with three 15 ml portions of ethyl acetate. The organic layers were combined, dried over anhydrous sodium sulfate, and concentrated under reduced pressure. The residue was subjected to silica gel column chromatography (1 g; ethyl acetate:hexane = 1:1 → 1:10 gradient) to give 2.8 mg (95 %) of (-)-$PGE_1$ methyl ester.

The IR and $^1H$ NMR spectra of the product completely agreed with those of the authentic sample.

Example 46

Synthesis of prostaglandin $F_{1\alpha}$ methyl ester 11,15-bis(t-butyldimethylsilyl)ether:-

14.29 mg (0.024 mmol) of 5,6-dehydroprostaglandin $F_{2\alpha}$ methyl ester 11,15-bis(t-butyldimethylsilyl) ether was dissolved in 4 ml of benzene-hexane (1:1), and 32 mg of synthetic quinoline and 24 mg of 5 % $Pd\text{-}BaSO_4$ were added. The mixture was stirred at 40 °C for 2.7 hours in an atmosphere of hydrogen. The catalyst was removed by filtration, and the filtrate was washed with ethyl acetate and concentrated under reduced pressure. The residue was allowed to stand overnight under reduced pressure produced by a vacuum pump, and then subjected to column chromatography ($SiO_2$ impregnated with $AgNO_3$;

ethyl acetate:hexane = 1:10) to give 8 mg (60 %) of prostaglandin $F_{1\alpha}$ methyl ester 11,15-bis(t-butyldimethylsilyl)ether.

The spectral data of the resulting product are as follows:-

IR ($CDCl_3$): 3620 - 3200, 1730 $cm^{-1}$.

Example 47

Synthesis of prostaglandin $F_{1\alpha}$ methyl ester:-

14.4 mg of prostaglandin $F_{1\alpha}$ methyl ester 11,15-bis(t-butyldimethylsilyl)ether was dissolved in the mixture of 1 ml of acetic acid, 0.33 ml of water and 0.1 ml of THF. The mixtrure was stirred at 60 $^{o}C$ for 1.5 hours. A toluene was added, and the reaction mixture was concentrated. The residue was subjected to silica gel column chromatography (3 g; ethyl acetate:hexane = 1:1 → 1:0, gradient) to obtain 6.8 mg (76 %) of (+)-$PGF_{1\alpha}$methyl ester.

The spectral data of the product are as follows:-

IR ($CDCl_3$): 3630 - 3200, 1732, 970 $cm^{-1}$.

$^{1}H$ NMR ($CDCl_3$) $\delta$:

0.89 (t, 3, J=6Hz, $CH_3$),
1.1 - 3.0 (m, 27, $CH_2$ x 11, CH x 2, OH x 3),
3.66 (s, 3, $OCH_3$), 4.05 (br, 3, CHO x 3),
5.48 (m, 2, vinyl).

$^{13}C$ NMR ($CDCl_3$) $\delta$:

13.6, 22.4, 24.8, 25.0, 27.9(2C), 28.9, 31.7, 34.0, 37.5, 43.3, 50.7, 51.0, 56.4, 72.7, 73.5, 78.5, 132.5, 134.7, 173.8.

$[\alpha]_D^{21}$: +29.3$^{o}$, (C=0.296, $CH_3OH$).

## CLAIMS

1. A process for the production of prostaglandins E or F of formula (I)

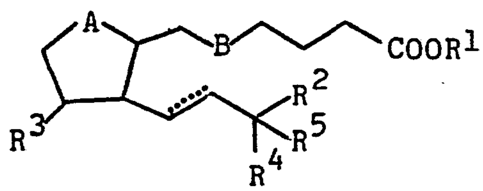

........... (I)

wherein

$R^1$ represents a hydrogen atom, a $C_{1-10}$ alkyl group or a tri($C_{1-7}$ hydrocarbon)silyl group,

$R^2$ represents an alkyl group having 1 to 10 carbon atoms which may be substituted, or a 5- or 6-membered cycloalkyl group which may be substituted,

$R^5$ represents a hydrogen atom or a methyl group,

$R^3$ and $R^4$ are identical or different and each represents a hydrogen atom, a hydroxyl group or a protected hydroxyl group,

the symbol ⋯ represents a single or double bond,

A represents a carbonyl group (>C=O) or a hydroxymethylene group (>CH-OH), and

B represents $-CH_2CH_2$, -CH=CH- or -C≡C-,

characterised by subjecting a 7-(thiocarboacyloxy)-prostaglandin of formula (II)

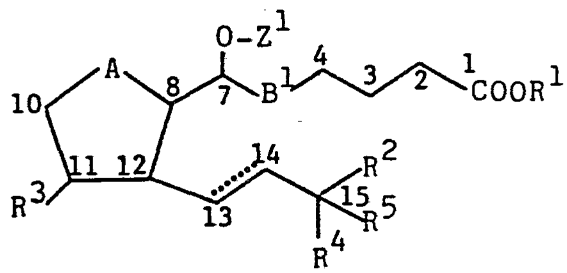

....... (II)

wherein

$R^1$, $R^2$, $R^3$, $R^4$, $R^5$, A and the symbol ⋯⋯

are as defined,

$B^1$ represents -CH=CH- or -C≡C-, and

$Z^1$ represents a thiocarboacyl group,

to a selective deoxygenation reaction, and thereafter, as required, subjecting the reaction product to reduction, deprotection and/or hydrolysis.

2. A process according to claim 1 wherein a prostaglandin E of formula (I), wherein A is a carbonyl group, is produced by subjecting a 7-(thiocarboacyloxy)-prostaglandin E of formula (II), wherein A is a carbonyl group, to a selective deoxygenation reaction, and thereafter, as required, subjecting the reaction product to selective reduction of the unsaturated bond ($B^1$) between the 5- and 6-positions, deprotection, and/or hydrolysis.

3. A process according to claim 1 wherein a prostaglandin F of formula (I), wherein A is a hydroxymethylene group, is produced by subjecting a 7-(thiocarboacyloxy)prostaglandin F of formula (II), wherein A is a hydroxymethylene group, to selective deoxygenation reaction, and thereafter, as required, subjecting the reaction product to selective reduction

of the unsaturated linkage ($B^1$) between the 5- and 6-positions, deprotection, and/or hydrolysis.

4. A process according to claim 1 for producing a prostaglandin F as defined in claim 3, which comprises subjecting a 7-(thiocarboacyloxy)prostaglandin E as defined in claim 2 to a selective deoxygenation reaction, reducing the oxo group (=O) at the 9-position, and thereafter, as required, subjecting the reaction product to selective reduction of the unsaturated bond ($B^1$) between the 5- and 6-positions, deprotection, and/or hydrolysis.

5. A process according to any one of claims 1 to 4 wherein the selective deoxygenation reaction is carried out in the presence of an organic tin compound of the formula $(R^6)_3SnH$ wherein $R^6$ represents a $C_{1-10}$ alkyl group or a phenyl group.

6. A process according to claim 5 wherein the selective deoxygenation reaction is carried out in the presence of a radical generator.

7. A process according to any one of the preceding claims wherein when $R^3$ and/or $R^4$ in formula (I) represents a protected hydroxyl group, the protected hydroxyl group is a tri-($C_{1-7}$ hydrocarbon)silyl group or a group capable of forming an acetal linkage with the oxygen atom of the hydroxyl group.

8. A 7-(thiocarboacyloxy)prostaglandin of formula (II)'

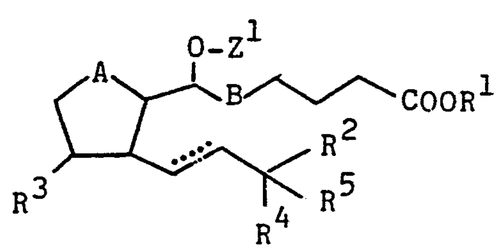

........ (II)'

wherein $R^1$, $R^2$, $R^3$, $R^4$, $R^5$, A, B, $Z^1$ and the symbol .... are as defined in claim 1.

9. A 7-(thiocarboacyloxy)prostaglandin according to claim 8 wherein $R^1$ is a hydrogen atom or a $C_{1-6}$ alkyl group, $R^2$ is an unsubstituted $C_{1-10}$ alkyl group, a $C_{1-10}$ alkyl group substituted by phenoxy, trifluoromethyl or trifluoromethylphenoxy, an unsubstituted 5- or 6-membered cycloalkyl group, or a 5- or 6-membered cycloalkyl group substituted by $C_{1-6}$ alkyl, trifluoromethyl, phenoxy or trifluoromethylphenoxy, $R^3$ and $R^4$ are identical or different and each represents a hydrogen atom, a hydroxyl group, a tri($C_{1-7}$ hydrocarbon)silyl group, or a group capable of forming an acetal linkage with the oxygen atom of the hydroxyl group and $Z^1$ is a thiocarboacyl group of the formula

$$-\underset{\underset{S}{\|}}{C}-X-G$$

wherein X represents a bond, an oxygen atom, a sulfur atom or -NHCO-, and G represents a substituted or unsubstituted $C_{1-6}$ alkyl, phenyl or imidazolyl group.

10. A 7-(carboacyloxy)prostaglandin of formula (III)

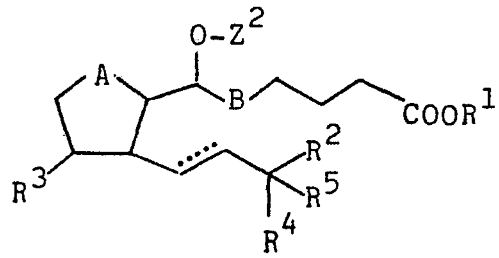

......... (III)

wherein $R^1$, $R^2$, $R^3$, $R^4$, $R^5$, A, B and the symbol .... are as defined in claim 1 and $Z^2$ represents a carboacyl group.

11. A 7-(carboacyloxy)prostaglandin according to claim 10 wherein $R^1$, $R^2$, $R^3$ and $R^4$ are as defined in claim 9 and $Z^2$ represents a carboacyl group of the formula $-\underset{\underset{O}{\|}}{C}-C_{1-10}$ alkyl.